# EUROPEAN PATENT APPLICATION

(11) **EP 3 872 182 A1**
(43) Date of publication of application: **01.09.2021**
(21) Application number: 20305210.5
(22) Date of filing: 28.02.2020
(51) Int. Cl.: C12N 15/82

(54) **RECOMBINANT MICROALGAE ABLE TO PRODUCE PEPTIDES, POLYPEPTIDES OR PROTEINS OF COLLAGEN, ELASTIN AND THEIR DERIVATIVES IN THE CHLOROPLAST OF MICROALGAE AND ASSOCIATED METHOD THEREOF**

(71) Applicant: Alganelle, 73370 Bourget Du Lac (FR)
(72) Inventor: TISSOT-LECUELLE, Ghislaine, 73290 LA MOTTE SERVOLEX (FR)
(74) Representative: Lavoix

(57) **Abstract**

The present invention concerns a recombinant microalgae comprising a nucleic acid sequence encoding a recombinant protein, polypeptide or peptide comprising repeat units of amino acids, said recombinant protein, polypeptide or peptide being chosen from collagen, elastin and their derivatives, and said nucleic acid sequence being located in the chloroplast of microalgae. It further relates to a method for producing a recombinant protein, polypeptide or peptide comprising repeat units of amino acids in the chloroplast of microalgae, said recombinant protein, polypeptide or peptide being chosen from collagen, elastin and their derivatives wherein said method comprises transforming the chloroplast genome of a microalgae with a nucleic acid sequence encoding said recombinant protein, polypeptide or peptide.

## Description

The present invention concerns a recombinant microalgae comprising a nucleic acid sequence encoding a recombinant protein, polypeptide or peptide comprising repeat units of amino acids, said recombinant protein, polypeptide or peptide being chosen from collagen, elastin and their derivatives, said nucleic acid sequence being located in the chloroplast genome of microalgae. It further relates to a method for producing a recombinant protein, polypeptide or peptide comprising repeat units of amino acids in the chloroplast of microalgae, said recombinant protein, polypeptide or peptide being chosen from collagen, elastin and their derivatives wherein said method comprises the chloroplast genome transformation of a microalgae with a nucleic acid sequence encoding said recombinant protein, polypeptide or peptide.

In recent years, the demand for recombinant protein is increasing more and more because of their high value applications in broad range industries as in personal care, cosmetics, healthcare, tissue engineering, biomaterials, agriculture and paper industries. Numerous examples of commercial pharmaceutical proteins produced in various recombinant systems have been launched, as for instance, insulin, human growth hormone, erythropoietin and interferon.

Additionally, large quantities of proteins and peptides are needed for these various industrial applications.

The most current industrial expression systems include the bacteria *E. coli,* the yeast (*S. cerevisiae* and *P. pastoris*) and mammalian cell lines. Emerging technologies are insect cell cultures, plants and microalgae.

However, the expression of recombinant peptides and proteins is still limited, as large efforts are required in order to obtain the desired peptides and proteins with a native conformation, in high amounts and high purity. Even a current bacterial system such as *E. coli* has limitations at expressing recombinant peptides/polypeptides/proteins. Indeed, formation of insoluble aggregates (or inclusion body) arises due to the lack of sophisticated machinery to perform posttranslational modifications, as for instance disulfide bound formation or glycosylations. This results in poor solubility of the protein of interest and/or in the absence of protein activity.

Interest in microalgae as an alternative platform for recombinant protein production has been gaining in recent years.

Recombinant algae offer several advantages over the other recombinant protein production platforms. Microalgae are photosynthetic unicellular microorganisms with low nutriment requirements to grow. They are capable of photoautotrophic, mixotrophic or heterotrophic growth. The cost of protein production in algae is much lower than other production systems in photoauxotrophic growth. Proteins purified from algae, as from plant, should be free from toxins and viral agents that may be present in preparations from bacteria or mammalian cell culture. Indeed, several microalgae species have the GRAS status (Generally Recognized As Safe) Granted by the FDA, as for instance for microalgae, *Chlorella vulgaris*, *Chlorella protothecoides* S106, *Dunaliella bardawil*, *Chlamydomonas reinhardtii* and for cyanobacteria *Arthrospira plantesis.*

As in transgenic plants, algae have been engineered, to express recombinant genes from both the nuclear and chloroplast genomes.

In addition, recombinant synthesis of peptides and polypeptides composed of repeat units of specific amino acid sequence is difficult because the DNA sequences encoding the peptides or polypeptides are often subject to genetic recombination resulting in genetic instability and leading often to the production of proteins smaller than the native one.

Recombinant production of relatively small peptides can be also challengeous because they can self-assemble or be subject to proteolytic degradation.

Moreover, in contrast to plant expression system, algae are robust industrial chassis with competitive production costs reachable at industrial scale in reproducible, sterile and well-controlled production conditions within photobioreactors and fermenters or in a single use wave bag. In addition, they can secrete recombinant proteins outside the cell and thus in the culture media, simplifying the subsequent purification steps. Algae have no seasonality and don't used arable land.

The development of chloroplast transformation in algae for the production of proteins of interest is more recent than in plants and requires improvement. In fact, the production yield of recombinant protein are typically between 0.5 and 5% of the total soluble proteins in *Chlamydomonas reinhardtii* chloroplast, which is still low in comparison to established microbial platform.

In addition, some mammalian proteins are not easily expressed (Rasala *et al.*, 2010).

The inventors of the present invention have surprisingly been able to produce, in the chloroplast of microalgae, a recombinant protein, polypeptide or peptide comprising several repeat units of amino acids, said recombinant protein, polypeptide or peptide being chosen from collagen, elastin and their derivatives.

Indeed, homologous recombination phenomenon between similar or identical sequences being highly efficiency in the chloroplast genome, transgene with repeat sequences could have been very instable.

By using said method, endogenous disulfide bond formation which is essential for protein, peptide and polypeptide stability and activity, and increased accumulation of the protein, peptide and polypeptide, is allowed.

The present invention thus relates to a recombinant microalgae comprising a nucleic acid sequence encoding a recombinant protein, polypeptide or peptide comprising repeat units of amino acids, said protein, polypeptide or peptide being chosen from collagen, elastin and their derivatives, and said nucleic acid sequence being located in the chloroplast genome of microalgae.

The present invention also relates to the use of said recombinant algae, for producing a recombinant protein, polypeptide or peptide comprising repeat units of amino acids, said protein, polypeptide or peptide being chosen from collagen, elastin and their derivatives.

The present invention further relates to a method for producing a recombinant protein, polypeptide or peptide comprising repeat units of amino acids in the chloroplast of microalgae, said recombinant protein, polypeptide or peptide being chosen from collagen, elastin and their derivatives, wherein said method comprises transforming the chloroplast genome of microalgae with a nucleic acid sequence encoding said recombinant protein, polypeptide or peptide.

In particular, said method comprises:
i) providing a nucleic acid sequence encoding said recombinant protein, polypeptide or peptide;
(ii) introducing the nucleic acid sequence according to (i) into an expression vector which is capable of expressing the nucleic acid sequence in microalgae host cell; and
(iii) transforming the chloroplast genome of microalgae host cell by the expression vector.

In particular, said method further comprises:
(iv) identifying the transformed microalgae host cell;
(v) characterizing the microalgae host cell for the production of recombinant protein, polypeptide or peptide expressed from said nucleic acid sequence;
(vi) extracting the recombinant protein, polypeptide, or peptide; and optionally
(vii) purifying the recombinant protein, polypeptide, or peptide.

More particularly, the method according to the invention allows to increase accumulation and/or stability and/or solubility and/or folding and/or activity of recombinant peptides, polypeptides or proteins comprising repeat units of amino acids in the chloroplast of microalgae.

In particular, said method further comprises a step (viii) further to step (vii) in which the polypeptide is cleaved to allow the release of peptide units.

Said cleavage can be carried out by any method known by the man skilled in the art such as the use of suitable endoproteinase.

In one embodiment, the recombinant protein, polypeptide or peptide obtained by the method according to the invention are chemically modified at their N- or C-terminus, for example by adding a palmitoyl group, an hydroxyl group, an alkoyl chain (i.e. an alkyl chain comprising an hydroxyl group), or a biotinyl group.

By "recombinant peptide/polypeptide/protein" is meant in the art, and in the context of the present invention, an exogenous peptide/polypeptide/protein expressed from a recombinant gene (or recombinant nucleic acid sequence) i.e. an exogenous gene (or exogenous nucleic acid sequence) being from a different species (heterologous) or from the same species (homologous).

By "recombinant microalgae" is meant a microalgae comprising a nucleic acid sequence encoding a recombinant protein, polypeptide or peptide. In the context of the invention, the recombinant microalgae is transformed as further detailed below.

By "peptides", "polypeptides", "proteins" is meant the meaning commonly understood by a person skilled in the art to which this invention belongs. In particular, peptides, polypeptides and proteins are amino acid polymers linked *via* peptide (amide) bonds.

More particularly, proteins according to the invention have unique and stable three-dimensional structure and are composed of more than 50 amino acids, like proteins of 54, 60, 66, 72, 75, 78, 84, 90, 96, 100, 102, 108, 114, 120, 150, 180, 200, 300, 350 or more amino acids; peptides according to the invention are of short oligopeptides, for examples peptides of 2 to 10 amino acids, like peptides of 4, 5, 6, 7, 8, 9 or 10 amino acids; polypeptides according to classical meaning can be composed of 11 to 50 amino acids, like polypeptides of 11, 12, 15, 18, 20, 24, 25, 30, 35, 36, 40, 42, 45, 48, or 50 amino acids; but in the context of the invention, polypeptides are a repetition of n units of identical or different amino acid sequence, or a repetition of n units of identical or different peptide, n being from 2 to 400, in particular from 2 to 100.

In the context of the invention, a recombinant protein, polypeptide or peptide according to the invention is a recombinant protein, polypeptide or peptide chosen from collagen, elastin and their derivatives, comprising repeat units of amino acids.

Elastin is a major structural protein of the extracellular matrix. It is present in the connective tissue of all vertebrates providing elasticity to tissues. Elastin is firstly synthetized as soluble monomer precursors, tropoelastin, which is subsequently assembled into the mature elastin, a stable polymeric structure.

This protein is well known in the art. The amino acid sequence of elastin and tropoelastin contains short repetitive amino acid motifs and numerous hydrophobic residues. During the aging process or after exposure to UVB-irradiation or in pathological processes, elastin is degraded into short peptides, named "elastin peptides" which play the role of signal peptides promoting for instance cell proliferation. These elastin peptides are part of matricins peptides.

Elastin peptides are considered as building blocks found in the natural elastin and have a short amino acid sequence.

Examples of elastin peptides according to the invention are pentapeptides: KGGVG (SEQ ID N°1), VGGVG (SEQ ID N°2), GVGVP (SEQ ID N°3), VPGXG (X being V, I, or K) (SEQ ID N°4), hexapeptides: VGVAPG (SEQ ID N°5), heptapeptides: LGAGGAG (SEQ ID N°6) or nonapeptides: LGAGGAGVL (SEQ ID N°7).

In the context of the invention, a recombinant peptide, polypeptide or protein consisting in repeated units of elastin, consists in repeated units (identical or different) of peptides of elastin and in particular of those (identical or different) of SEQ ID N°1 to 7.

"Derivatives" of elastin protein/polypeptide/peptide covers both elastin like proteins/polypeptides/peptides and proteins/polypeptides/peptides in which the amino acid sequence of the native elastin protein/polypeptide/peptide is mutated or contains one amino acid or more at its N- or C-terminus. The supplementary amino acids can be any amino acids. Derivatives of elastin protein/polypeptide/peptide also covers derivatives of elastin like proteins/polypeptides/peptides.

The peptides, polypeptides and peptides of elastin and their derivatives, according to the invention, thus also cover elastin like peptides, polypeptides, peptides and their derivatives.

Elastin like proteins, polypeptides or peptides (ELP) are synthetic molecules comprising mainly several fold repeated units of peptides or derivatives.

By "mutated" peptide, polypeptide or protein is meant that the nucleic or amino acid sequences of the mutated peptide, polypeptide or protein contains one or more mutations. These mutations include deletions, substitutions, insertions and /or cleavage of one or more nucleic acids or amino acids.

There are many variants of elastin-like polypeptides, comprising repeated units of different elastin peptides, such as those of SEQ ID N°1 to 7.

As an example, the elastin peptide described in this invention is the hexapeptide of SEQ ID N°5 (VGVAPG) or its derivatives.

The elastin like polypeptides or proteins described in this invention can thus, for example, comprise a n-fold repeat of this hexapeptide :(VGVAPG)ₙ, or of its derivatives. The number n can be for example from 2 to 200, with a preference from 2 to 100.

In particular, derivatives of the peptide of SEQ ID N°5 contain the VGVAPG sequence with one or more amino acids at its N- and/or C-terminus.

In particular, the supplementary amino acids in the derivatives of the peptide of SEQ ID N°5 are an aspartic acid or a glutamic acid.

More particularly, said derivatives can be of SEQ ID N°8 (VGVAPGD) or SEQ ID N°9 (VGVAPGE).

Still particularly the elastin like polypeptide described in this invention comprises a repeat of the hexapeptide of SEQ ID N°5, more particularly a 4-fold of this hexapeptide (named in the present invention ELP4 and being of SEQ ID N°81: VGVAPGVGVAPGVGVAPGVGVAPG). Another example of elastin like polypeptide derivative described in this invention comprises a repeat of the hexapeptide of SEQ ID N°9, more particularly a 4-fold of this hexapeptide (named in the present invention ELPE4 and being of SEQ ID N°80: VGVAPGEVGVAPGEVGVAPGEVGVAPGE).

An interesting feature of ELPs is that they can self-aggregates with an increase of temperature or a modification in pH and ionic strength. This feature can facilitated the purification step of ELPs after production in host cells.

The collagens are a superfamily of structurally related proteins that constitute essential building elements of connective tissues and participate to many biological functions in animals. These proteins exhibit a characteristic triple-helix tertiary structure resulting from the association of three polypeptide chains comprising repeated amino acid sequence Gly-X-Y (or GXY) where the amino acids X and Y are frequently proline or 4-hydroxyproline involved in the triple helix formation.

In humans, there are at least 27 different types of collagens found in different tissues (as bones, bones, skin, tendon, blood vessels, eyes, etc....).

In the context of the invention, a recombinant peptide, polypeptide or protein of collagen consists in repeated units of collagen motifs, i.e in repeated units of the sequence GXY.

Derivatives" of collagen protein/polypeptide/peptide covers both collagen like proteins/polypeptides/peptides and proteins/polypeptides/peptides in which the amino acid sequence of the native collagen protein/polypeptide/peptide is mutated or contains one amino acid or more at its N- or C-terminus. This supplementary amino-acids can be any amino acids. Derivatives of collagen protein/polypeptide/peptide also covers derivatives of collagen like proteins/polypeptides/peptides.

Collagen like proteins, peptides or polypeptides comprise mainly several fold repeated units of collagen motifs, i.e of repeated units of the sequence GXY (whereas peptide, polypeptide or protein of collagen contain only those repeated units).

Said repeated units are called "collagen like domain" and are capable to form triple helix (as collagen proteins), as for instance, C-type lectins (collectins), which participate in the host defense mechanism.

Moreover, screening of genome databases of gene encoding collagen-like sequences containing repeated GXY motifs have identified gene in the genomes of bacteria and phages. However, these organisms seem to lack proline hydroxylases. Recent studies have shown that two recently identified streptococcal collagen-like proteins, Scl1 and Scl2 as models are capable of forming a stable triple helix without hydroxylation of the proline residues.

In particular, collagen like proteins, peptides or polypeptides are and/or derive (of one or more amino acids) in particular in the present invention from microalgae, as for instance, from the CCMP2712 protein from *Guillardia theta* ((called GtCLP).

In one embodiment, derivatives according to the invention consists in an amino acid sequence at least 80% identical to the amino acid sequence of the recombinant peptide, polypeptide or protein according to the invention.

By "an amino acid sequence at least 80% identical" is meant in particular, an amino acid sequence 81, 82, 85, 90, 91, 92, 93, 94, 95, 96, 97, 98 or 99% identical. By an amino acid sequence at least 95% "identical" to a query amino acid sequence of the present invention, it is intended that the amino acid sequence of the subject peptide, polypeptide or protein is identical to the query sequence except that the subject amino acid sequence may include up to five amino acids alterations per each 100 amino acids of the query amino acids sequence. In other words, to obtain an amino acids sequence at least 95% identical to a query amino acid sequence, up to 5% (5 of 100) of the amino acid residues in the subject sequence may be inserted, deleted, or substituted with another amino acid.

In the frame of the present application, the percentage of identity is calculated using a global alignment (*i.e.* the two sequences are compared over their entire length). Methods for comparing the identity of two or more sequences are well known in the art. The « needle » program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is for example available on the ebi.ac.uk world wide web site. The percentage of identity in accordance with the invention is preferably calculated using the EMBOSS::needle (global) program with a "Gap Open" parameter equal to 10.0, a "Gap Extend" parameter equal to 0.5, and a Blosum62 matrix.

Amino acid sequences "at least 80%, 85%, 90%, 95% or 99% identical" to a reference sequence may comprise mutations such as deletions, insertions and/or substitutions compared to the reference sequence. In case of substitutions, amino acid sequences at least 80%, 85%, 90%, 95% or 99% identical to a reference sequence may correspond to a homologous sequence derived from another species than the reference sequence. In another preferred embodiment, the substitution preferably corresponds to a conservative substitution as indicated in the table below.

| Conservative substitutions | Type of Amino Acid |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

According to the invention, a nucleic acid sequence encoding said recombinant protein, polypeptide or peptide is a nucleic acid sequence encoding the proteins, peptides or polypeptides mentioned above.

In particular, for proteins, a nucleic acid sequence encoding elastin protein, elastin like proteins, collagen or collagen like proteins is contemplated, in particular, a collagen like protein is contemplated.

For example, the following nucleic acid sequence encoding a collagen like protein can be cited, *3f-tv-Gtclp-tv-ha* (SEQ ID N°10).

In particular, for peptides, a nucleic acid sequence encoding collagen like peptides, collagen peptides, elastin peptides and elastin like peptides is contemplated; more particularly a nucleic acid sequence encoding a collagen like peptide or elastin like peptide.

For example, the following nucleic acid sequences can be cited: SEQ ID N°11 (GTAGGTGTAGCTCCTGGT), SEQ ID N°12 (GTTGGTGTTGCTCCTGGA), SEQ ID N°13 (GTAGGTGTTGCTCCAGGT) and SEQ ID N°14 (GTGGGTGTAGCTCCTGGT), all these nucleic sequences encoding the elastin peptide VGVAPG previously mentioned. Other nucleic acid sequences can be cited as examples such as SEQ ID N°15 (GTAGGTGTAGCTCCTGGTGAA), SEQ ID N°16 (GTTGGTGTTGCTCCTGGAGAA), SEQ ID N°17 (GTAGGTGTTGCTCCAGGTGAA) and SEQ ID N°18 (GTGGGTGTAGCTCCTGGTGAA), all encoding the elastin peptide derivative VGVAPGE previously mentioned.

In particular, for polypeptides, a nucleic acid sequence encoding collagen like polypeptides, collagen polypeptides, elastin polypeptides and elastin like polypeptides are contemplated; more particularly a nucleic acid sequence encoding collagen polypeptide or an elastin like polypeptide.

For example, the following nucleic acid sequences can be cited *ha-sp-3f-Gtccld-3ha* (SEQ ID N°19), *ha-sp-3f-Gtccld* (SEQ ID N°20) and *ha-sp-3f-Gtcld* (SEQ ID N°21), *elp4* (SEQ ID N°22), and *elpe4* (SEQ ID N°23).

Still particularly, nucleic acid sequence encoding derivatives according to the invention consists in a nucleic acid sequence at least 80% identical to the nucleic acid sequence encoding the recombinant peptide, polypeptide or protein according to the invention. By "a nucleic acid sequence at least 80% is meant in particular, a nucleic acid sequence 81, 82, 85, 90, 91, 92, 93, 94, 95, 96, 97, 98 or 99% identical. For example, a nucleic acid sequence 95% "identical" to a query sequence of the present invention, is intended to mean that the sequence of the polynucleotide is identical to the query sequence except that the sequence may include up to five nucleotide alterations per each 100 nucleotides of the query sequence. In other words, to obtain a polynucleotide having a sequence at least 95% identical to a query sequence, up to 5% (5 of 100) of the nucleotides of the sequence may be inserted, deleted, or substituted with another nucleotide. In other terms, the sequences should be compared on their entire length (i.e. by preparing a global alignment). For example, a first polynucleotide of 100 nt (nucleotides) that is comprised within a second polynucleotide of 200 nt is 50% identical to said second polynucleotide. The needle program, which uses the Needleman-Wunsch global alignment algorithm (Needleman and Wunsch, 1970, A general method applicable to the search for similarities in the amino acid sequence of two proteins, J. Mol. Biol. 48:443-453) to find the optimum alignment (including gaps) of two sequences when considering their entire length, may for example be used. Preferably, the percentage of identity in accordance with the invention is calculated using the needle program with a "Gap open" parameter equal to 10.0, a "Gap Extend" parameter equal to 0.5, and a Blosum 62 matrix. The needle program is for example available on the ebi.ac.uk World Wide Web site.

In one embodiment, the nucleic acid sequence encoding the protein, polypeptide or peptide comprising repeat units of amino acids according to the invention is codon optimized for expression in the chloroplast genome of the microalgae host cell.

As mentioned above, the nucleic acid sequence according to the invention is introduced into an expression vector which is capable of expressing the nucleic acid sequence.

By "introduction" is meant cloning the nucleic acid sequence encoding the recombinant protein/polypeptide/peptide inside the expression vector with the methods well known by the skilled man and in the way to lead to the expression of this nucleic acid sequence.

"Expression vector" or "transformation vector" or "recombinant DNA construct", or similar terms, are defined herein as DNA sequences that are required for the transcription of recombinant genes and the translation of their mRNAs in the microalgae host cells. "Expression vector" comprises one or more expression cassettes for the expression of recombinant genes (one or more genes encoding the protein, peptide or polypeptide of interest and often selectable markers). In the case of chloroplast genome transformation, expression vectors also contain homologous recombination regions for the integration of expression cassettes inside the chloroplast genome.

In the context of the invention, the expression vector can be in particular a circular molecule with a plasmid backbone containing the two homologous recombination regions and flanking the expressions cassettes, or a linearized molecule corresponding to the expression vector linearized by enzymatic digestion or to a PCR fragment containing only the expression cassettes flanked by the two homologous recombination regions.

In particular, expression vectors of the invention comprise at least one expression cassette and are for example vectors pCO86, pCO96, pCO26, pCO28, pLA01, pLA02, pAL03 or pAL04.

"Expression cassette" contains a coding sequence fused operationally to one or more regulatory elements or regulatory sequences, as for instance, fused at its 5'end to a promoter and/or 5'UTR and/or at its 3'end to a 3'UTR.

The "coding sequence" is the portion of a gene and of its corresponding transcribed mRNA which is translated into the recombinant protein/polypeptide/peptide. The coding sequence includes, for example and a translation initiation control sequence and a stop codon. In some embodiment, the expression cassette can contain a polycistron composed of more than one coding sequence encoding several proteins under the control of only one promoter/5'UTR and 3'UTR.

Said expression cassettes are flanked by left (LHRR) and right (RHRR) endogenous sequences identical to those surrounding the targeted integration site into the chloroplast genome. These left (LHRR) and right (RHRR) homologous regions allow the integration of expression cassettes after homologous recombination exchange between the regions of homology.

Homologous recombination is the ability of complementary DNA sequences to align and exchange regions of homology. Transgenic DNA ("donor") containing sequences homologous to the genomic sequences being targeted ("template") is introduced into the organism and then undergoes recombination into the genome at the site of the corresponding genomic homologous sequences.

By its very nature homologous recombination is a precise gene targeting event, hence, most transgenic lines generated with the same targeting sequence will be essentially identical in terms of phenotype, necessitating the screening of far fewer transformation events.

In the case of chloroplast genome transformation of microalgae, the integration of expression cassettes inside the chloroplast genome occurs after homologous recombination between the endogenous homologous sequences of the expression vector with the genome sequences identical or similar to those surrounding the targeted integration site into the chloroplast genome.

In the context of the present invention, different integration sites can be used, between the genes *rbcL* and *atpA,* or *psaB* and *trnG,* or *atpB* and *16S rDNA,* or *psaA exon3* and *trnE,* or *trnE* and *psbH* or *psbN* and *psbT,* or *psbB* and *trnD.*

In some embodiments, in order to enhance its accumulation, the recombinant protein or polypeptide or peptide can be fused to endogenous proteins, as for instance to the large subunit of ribulose bisphosphate carboxylase (Rubisco LSU). In this case, the promoter and 5'UTR will be those of the endogen *rbcL* gene, after homologous recombination of the transformation vector into the chloroplast genome.

The protein, peptide or polypeptide of interest will be further separated from RBCL either *in vivo* (using self-cleavable peptides) or *in vitro* (by site specific proteolysis), depending of the chosen processing system.

In one embodiment, said coding sequence of the expression cassette according to the invention also comprises a nucleic acid sequence encoding an epitope tag, in particular the Flag epitope Tag, more particularly the Flag epitope Tag repeat 3 times (3xFlag Tag), in order to identify and/or purify the recombinant protein, polypeptide or peptide.

In particular, said epitope Tag sequence is placed at the N-terminus of the protein, peptide or polypeptide. More particularly, another epitope Tag sequence can be placed at the C-terminus of the protein, peptide, or polypeptide according to the invention, alone or in addition to the one at the N-terminus and this, in order to monitor the release of the peptide/polypeptide/protein of interest to follow its cleavage, for example by an endoprotease.

Examples of epitope Tag sequences are Flag Tag (SEQ ID N°24: DYKDDDDK), 3xFlag Tag (SEQ ID N°25: DYKDDDDKDYKDDDDKDYKDDDDK), HA Tag (SEQ ID N°26: YPYDVPDYA), 3xHA Tag (SEQ ID N°27: YPYDVPDYAYPYDVPDYAYPYDVPDYA), His Tag (SEQ ID N°28: HHHHHH), which are described in the experimental part of the invention.

In one embodiment, said coding sequence in the expression cassettes comprises nucleic acid sequences encoding not only the said recombinant protein, polypeptide or peptide but also an amino acid sequence allowing the production of the said recombinant protein, polypeptide or peptide in specific cell compartment.

"Promoter" as used herein, refers to a nucleic acid control sequence that directs transcription of a nucleic acid.

"5'UTR" or 5' untranslated region (also known as a leader sequence or leader RNA) is the region of an mRNA that is directly upstream from the initiation codon. "3'UTR" or 3' untranslated region is the section of messenger RNA (mRNA) that immediately follows the translation termination codon.

5'UTRs and 3'UTRs are required for transcript (mRNA) stability and translation initiation.

For microalgae chloroplast expression, promoters, 5'UTRs and 3'UTRs that can be used in the context of the invention are for example: the promoters and 5'UTRs of the genes *psbD*, *psbA*, *psaA*, *atpA*, and *atpB*, the *16S rRNA* promoter (*Prrn*) promoter fused with a 5'UTR, the *psbA* 3' UTR, the *atpA* 3'UTR or the *rbcL* 3' UTR.

A 5'UTR from exogenous origin as for instance the 5'UTR of the gene *10L* of the bacteriophage T7 can be used also fused downstream a microalgae promoter. In particular, the nucleic acid sequence is operationally linked at its 5'end to the *Chlamydomonas reinhardtii 16S rRNA* promoter (*Prrn*).

Stable expression and translation of the nucleic acid sequence according to the present invention can for example be controlled by the promoter and 5'UTR from *psbD* and the *atpA* 3'UTR.

Also, in one embodiment of the recombinant microalgae or method according to the invention, nucleic acid sequence encoding a recombinant protein, polypeptide or peptide is operably linked to at least one regulatory sequence chosen from the *psbD* promoter and 5'UTR (SEQ ID N°29), or the *16S rRNA* promoter (*Prrn*) promoter fused with the *atpA* 5'UTR (SEQ ID N°30), the *psaA* promoter and 5'UTR, the *atpA* promoter and 5' UTR, the 3'UTRs from *atpA* (SEQ ID N°31) and *rbcL* (SEQ ID N°32).

In one embodiment, the promoter less gene encoding the protein, peptide or polypeptide of interest can be integrated after homologous recombination region inside the chloroplast genome just downstream a native promoter.

As mentioned above, the chloroplast genome of microalgae host cell is transformed by the expression vector. This genetic transformation of microalgae host cells, and more particularly the chloroplast genome of microalgae, by expression vector according to the invention can be carried out according to any suitable techniques well known by the man skilled in the art including, without limitations biolistics (Boynton et al., 1988; Goldschmidt-Clermont, 1991), electroporation (Fromm et al., Proc. Nat. Acad. Sci. (USA) (1985) 82:5824-5828 ; see Maruyama et al. (2004), Biotechnology Techniques 8:821-826), glass bead transformation (Purton *et al.*, revue), protoplasts treated with CaCl₂ and polyethylene glycol (PEG) (see Kim et al. (2002), Mar. Biotechnol. 4:63-73) or microinjection.

In particular, said transformation uses the helium gun bombardment technique of gold micro-projectiles complexed with transforming DNA.

To identify the microalgal transformants, a selectable marker gene may be used. Mention may be made for example of the *aadA* gene coding aminoglycoside 3"-adenylyltransferase and conferring the resistance to spectinomycin and streptomycin in the case of *Chlamydomonas reinhardtii* chloroplast transformation. In another embodiment, the selectable marker gene can be the *Acinetobacter baumannii aphA-6 Ab* gene encoding 3'-aminoglycoside phosphotransferase type VI and conferring the kanamycin resistance.

Chloroplast genome engineering can thus be performed using selectable maker gene conferring resistance to antibiotic or using rescue of photosynthetic mutant.

In particular, in one embodiment, the expression vector for chloroplastic genome transformation comprises two expression cassettes comprising the nucleic acid sequence encoding the recombinant protein, polypeptide or peptide according to the invention or the selectable marker gene.

More particularly, the expression vector for chloroplastic genome transformation comprises one expression cassette comprising the nucleic acid sequence encoding the recombinant protein, polypeptide or peptide according to the invention and one expression cassette comprising the *aadA* gene coding aminoglycoside 3"-adenylyltransferase.

In another embodiment of the invention, corresponding to the case of rescue of photosynthetic mutant sensitive to light, the expression vector comprises the wild type RHRR region which is deleted in the mutant strain. After homologous recombinations, the deleted region is restored in the genome of the photosynthetic mutant which is able then to growth under light.

In particular, the coding sequence of the expression cassette according to the invention also comprises a nucleic acid sequence encoding a signal peptide. By "signal peptide" (SP) is meant in the present invention an amino acid sequence placed at the N-terminus of a newly synthetized recombinant protein or polypeptide or peptide. This signal peptide should allow the translocation of the protein inside the lumen of chloroplast thylakoids and not in the chloroplast stroma. The signal peptide is cleaved after translocation across the thylakoid membranes.

For example, such signal peptide sequence is chosen from known protein translocated inside the lumen of thylakoids using either the twin-arginine protein translocation (Tat) pathway or the Sec pathway.

For instance, signal peptides can derive from algae proteins localized in the thylakoid lumen, as the signal peptide from the *Chlamydomonas reinhardtii* 16 and 23 kDa subunits of the oxygen-evolving complex of photosystem II, or the *Chlamydomonas reinhardtii* Rieske subunit of b₆f complex or the cryptophytes phycoerythrin alpha subunit (as example from *Guillardia theta*).

In particular, the Signal Peptide can be extracted from the sequence of the *E. coli TorA* gene encoding the Trimethylamine-N-oxide reductase 1 (UniProt number P33225) (SP; SEQ ID N°33: NNNDLFQASRRRFLAQLGGLTVAGMLGPSLLTPRRATAAQA; nucleic acid sequence encoding SP is SEQ ID N°34). This signal peptide used the Tat system. This amino acid sequence is cleaved from the protein after the translocation of the later one across the thylakoid membrane.

Other signal peptides can be used which don't leave supplementary amino acids at the N-terminus of the recombinant protein as in particular signal peptide from algae, and in particular from *Chlamydomonas reinhardtii.*

In one embodiment, the recombinant protein or polypeptide or peptide can be produced as a fusion protein.

The invention thus also relates to a recombinant microalgae or method according to the invention, wherein the nucleic acid sequence encoding a recombinant protein, polypeptide or peptide is fused operationally at its 5' or 3'end to a nucleic acid sequence encoding a carrier.

Fusion partners or carriers have been developed in recombinant protein production in order to increase accumulation yields, and/or solubility and/or folding and/or to facilitate protein purification. Fusion partners of different sizes (or molecular weight) have been used in various production systems in order to enhance protein solubility and accumulation (maltose-binding protein (MBP), glutathione-S-transferase (GST), thioredoxin, GB1, N-utilizing substance A (NusA), ubiquitin, small ubiquitin-like modifier (SUMO), Fh8) and to facilitate detection and purification (as for examples without limitation MBP, GST and small epitope Tag peptides as c-myc Tag, poly-histidine Tag (His Tag), Flag Tag, HA Tag. Another type of fusion Tags used for purification are stimulus response Tags (or environmentally responsive polypeptides) which allow precipitation of the fusion protein when stimulus as modification of temperature or solution ionic strength are adjusted.

In particular, a carrier according to the present invention is aprotinin.

The carrier and the recombinant protein, polypeptide or peptide are fused together to form a fusion protein.

By « aprotinin » is meant the basic trypsin inhibitor (BPTI), a small single-chain protein cross-linked by three disulfide bridges which consists of 58 amino acid residues with a molecular mass of 6.5 kDa and an isoelectric point of 10.9.

Said protein is well known by the man skilled in the art and is available commercially. It can for example be produced in recombinant systems such as plants (in cytoplasm by nuclear transformation (Pogue *et al.*, 2010), or in thylakoid lumens by chloroplast transformation (Tissot *et al*., 2008).

Its formula is C₂₈₄H₄₃₂N₈₄O₇₉S₇ and its molar mass 6511.51 g/mol g.mol⁻¹.

The amino acid sequence for aprotinin from *Bos Taurus* (bovine) is RPDFC LEPPY TGPCK ARIIR YFYNA KAGLC QTFVY GGCRA KRNNF KSAED CMRTC GGA (SEQ ID N°35). The nucleic acid sequence encoding this amino acid sequence is the SEQ ID N°36.

In the context of the present invention, the term "aprotinin" also covers chimeric aprotinin and mutated aprotinin.

By "chimeric aprotinin" is meant that aprotinin is connected at its N-terminus and/or at its C-terminus to an epitope Tag peptide(s) and/or signal peptide and/or protease recognition cleavage site.

The chimeric aprotinin can be for example the protein called HA-APRO (SEQ ID N°37), comprising aprotinin fused at its N-terminus to the HA epitope Tag or 3F-APRO (SEQ ID N°39) comprising aprotinin fused at its N-terminus to the 3xFlag epitope Tag (3F).

Other examples of chimeric aprotinin can be the protein called HA-SP-3F-FX-APRO (SEQ ID N°41 and 42) comprising aprotinin fused at its N-terminus to an amino acid sequence made of the HA epitope Tag (HA) followed by the signal peptide (SP), the 3xFlag epitope Tag (3F), and the cleavage site for Factor Xa (FX; IEGR), or the chimeric aprotinin called HA-SP-3F-APRO (SEQ ID N°43 and 44) comprising aprotinin fused at its N-terminus to the HA epitope Tag followed by the signal peptide SP and the 3xFlag epitope Tag (3F) or the chimeric aprotinin called HA-SP-APRO (SEQ ID N°45 and 46) comprising aprotinin fused at its N-terminus to the HA epitope Tag followed by the signal peptide SP.

By "mutated" aprotinin is meant that the nucleic or amino acid sequence of the "mutated" aprotinin contains one or more mutations in the nucleic or amino acid sequences of aprotinin or chimeric aprotinin. These mutations include deletions, substitutions, insertions and /or cleavage of one or more nucleic acids or amino acids.

Signal peptide is as previously described.

Other signal peptides can be used which doesn't leave supplementary amino acids at the N-terminus of the recombinant protein. If the signal peptide is cleaved after translocation into the lumen of chloroplast thyalkoïds (or across the thylakoid membranes), two other chimeric aprotinin can be produced *in vivo* 3F-APRO or 3F-FX-APRO (SEQ ID N°47).

In particular, according to the invention, the fusion partner is used to improve the accumulation and/or the stability of recombinant peptides, polypeptides and proteins.

Still particularly, and as mentioned above, said fusion protein also comprises cleavage sites recognized by a specific protease.

Cleavage sites recognized by specific proteases are well known of the man skilled in the art. They are used to separate the aprotinin from the recombinant protein, polypeptide or peptide of interest, in the case the carrier should be removed if it could interfere with the activity or the structure of said protein, polypeptide or peptide and thus with its uses.

In particular, said cleavage sites are an endoprotease and/or endoproteinase recognition sequence (or protease cleavage site or protease recognition site). More particularly, the sequence of said cleavage sites is placed between the two coding sequences (the one of aprotinin and the one of the recombinant protein, polypeptide or peptide of interest according to the invention).

The cleavage of the fusion protein can be performed either *in vivo* (in the recombinant host cell before extraction or when apply on the skin for cosmetic peptides) or *in vitro* after extraction and purification by adding protease.

Non limitative examples of proteases are Factor Xa (FX), Tobacco Edge Virus protease (TEV), enterokinase (EK), SUMO protease, Thrombin, Human Rhinovirus 3C Protease (HRV 3C), endoproteinase Arg-C, endoproteinase Asp-C, endoproteinase Asp-N, endoproteinase Lys-C, endoproteinase Glu-C, proteinase K, IgA-Protease, Trypsin, chymotrypsin and Thermolysin.

Self-cleavage peptides can also be used, as for example the Intein system (Yang *et al.*, 2003), the viral 2A system (Rasala *et al.*, 2012) or the site of the preferredoxin from *Chlamydomonas* (Muto *at al.*, 2009).

In one embodiment, a linker can be placed between aprotinin and the protease cleavage site. Linkers can be classified into three types: flexible, rigid and cleavable. The usual function of linkers is to fuse the two partners of the fusion protein (e.g. flexible linkers or rigid linkers) or to release them under specific conditions (cleavable linkers) or to provide other functions of the proteins in drug design such as improving of their biological activities or their targeted delivery.

In one embodiment of the present invention, the linker can also make the protease cleaving site more accessible to the enzyme if necessary.

In one embodiment, the flexible linker contains small, non-polar (e.g. Gly) or polar (e.g. Ser or Thr) amino acids. Examples of such linkers are given in Chen *et al*., 2013.

Flexible linkers according to the invention can be LG (SEQ ID N°49: RSGGGGSGGGGSGS) or LGM (SEQ ID N°50: RSGGGGSSGGGGGGSSRS).

When a fusion protein with a carrier is involved, step (vii) of the method according to the invention is a step of purification the fusion protein.

In that case, the method comprises optionally a step (viii) in which the fusion protein is cleaved.

Said cleavage can be carried out by any method known by the man skilled in the art such as the use of suitable protease to release the recombinant peptide, polypeptide or protein.

Said step (viii) is optionally followed by a purification step (ix) of the recombinant protein, polypeptide or peptide.

In particular, said method further comprises a step (viii') between step (viii) and step (ix), in which the polypeptide is cleaved to allow the release of peptide units.

Said cleavage can be carried out by any method known by the man skilled in the art such as the use of suitable endoproteinase

Characterization of the microalgae host cell producing the recombinant protein, polypeptide or peptide can be conducted by techniques known by the man skilled in the art, for example by PCR screening of the antibiotic resistant transformants or Western Blot analysis performed on total protein extracts.

Extraction of total proteins can be carried out using well known techniques (centrifugation, lysis, sonication, ...).

Identification of fusion or recombinant proteins can be carried out by Western blot using specific antibodies.

Purification can be carried out using well-known techniques. In one embodiment, it comprises an affinity chromatography and/or a step of separation of the peptide, polypeptide or protein according to the invention from the carrier (for example by enterokinase protease digestion) and/or a size exclusion chromatography.

In one embodiment, the step of affinity chromatography can be replaced by an ion exchange chromatography, less expensive for large scale purification.

According to the invention, "microalgae" is a eukarytotic microbial organism that contains a chloroplast or plastid, and optionally that is capable of performing photosynthesis, or a prokaryotic microbial organism (cyanobacteria) capable of performing photosynthesis.

In particular, said microalgae is chosen from the group consisting *Chlorophyta* (green algae), *Rhodophyta* (red algae), *Stramenopiles* (heterokonts), *Xanthophyceae* (yellow-green algae), *Glaucocystophyceae* (glaucocystophytes), *Chlorarachniophyceae* (chlorarachniophytes), *Euglenida* (euglenids), *Haptophyceae* (coccolithophorids), *Chrysophyceae* (golden algae), *Cryptophyta* (cryptomonads), *Dinophyceae* (dinoflagellates), *Haptophyceae* (coccolithophorids), *Bacillariophyta* (diatoms), *Eustigmatophyceae* (eustigmatophytes), *Raphidophyceae* (raphidophytes), *Scenedesmaceae, Phaeophyceae* (brown algae).

More particularly, said microalgae is chosen from the group consisting of *Chlamydomonas, Chlorella, Dunaliella, Haematococcus,* diatoms, *Scenedesmaceae, Tetraselmis*, *Ostreococcus*, *Porphyridium,* and *Nannochloropsis.*

Even more particularly said microalgae is chosen from the group consisting of *Chlamydomonas,* more particularly *Chlamydomonas reinhardtii,* even more particularly *Chlamydomonas reinhardtii 137c or a deficient strain as Chlamydomonas reinhardtii CW15.*

In particular, said microalgae is cultured in classic conditions known by the man skilled in the art. For example, *Chlamydomonas reinhardtii* is grown in TAP (Tris Acetate Phosphate) medium to mid-logarithmic phase (densities of approximately 1-2x10⁶ cell/ml), and/or at a temperature comprised between 23°C to 25°C (ideally 25°C), and/or on a rotary shaker in presence of constant light (70-150) µE/m²/s). The experimental part illustrates the conditions of culture.

All the embodiments mentioned in the context of the present invention can be combined.

The invention will be further illustrated by the following figures and examples.

### FIGURES

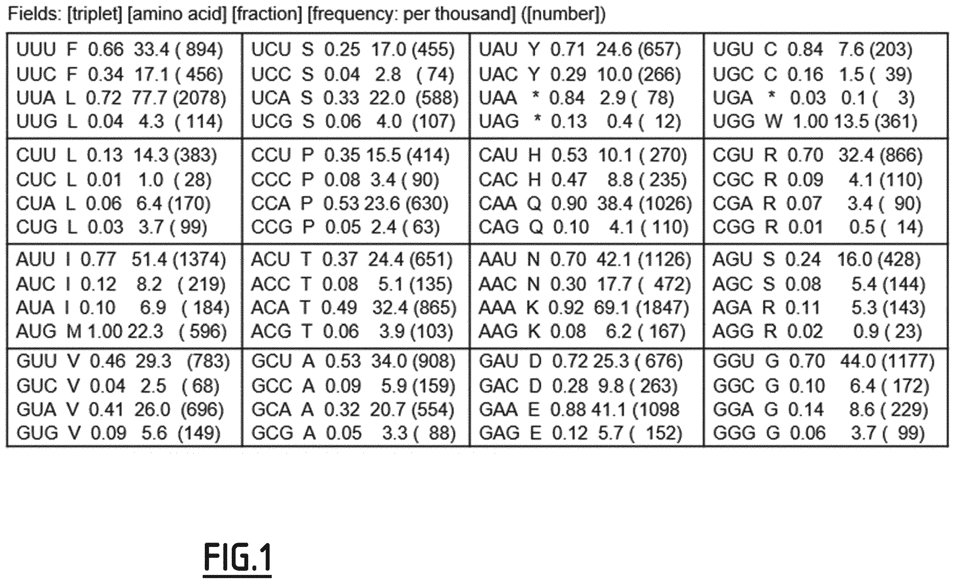

**Figure 1****:** Codon usage in the *Chlamydomonas reinhardtii* chloroplast genome

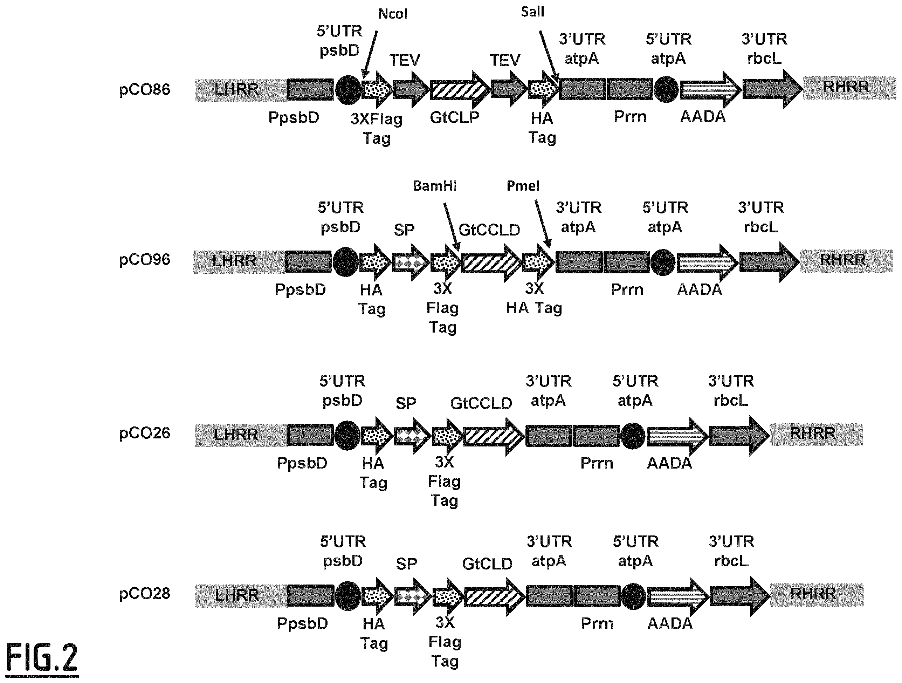

**Figure 2****:** Schematic presentation of the chloroplast transformation vectors for the production of *G. theta* CCMP2712 protein (GtCLP), the native and chimeric Collagen-Like Domains (GtCLD) of *G. theta* CCMP2712.

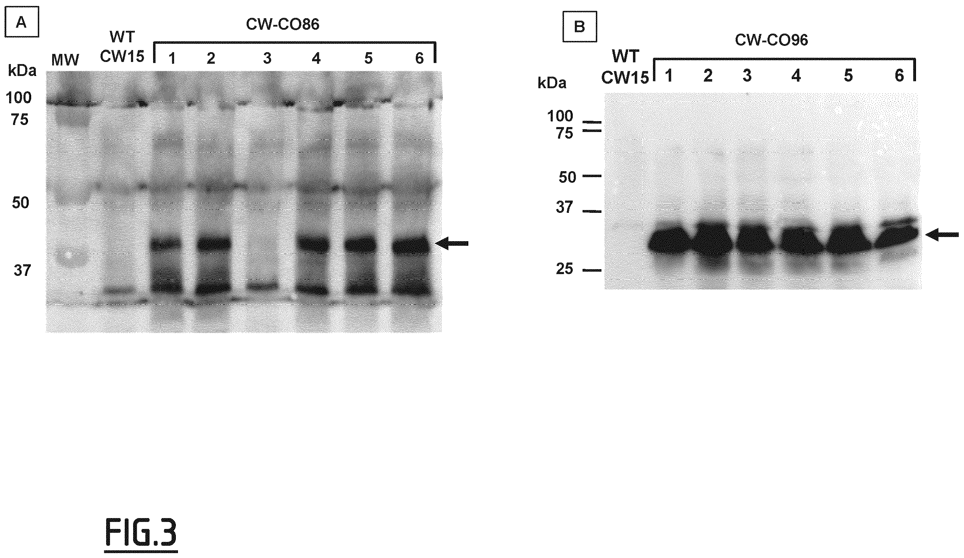

**Figure 3****:** Western blot analysis of independent algae transformants CW-CO86 and CW-CO96 expressing the genes of *G. theta* CCMP2712 protein (A) and the chimeric Collagen-Like domain of *G. Theta* CCMP2712 (B) from the algae chloroplast genome, using monoclonal anti-Flag M2 antibody. 100 µg of each total soluble protein samples extracted with SDS buffer lysis were separated on a 15% SDS polyacrylamide gel. MW: molecular weight standard. Arrows indicate the positions of recombinant proteins.

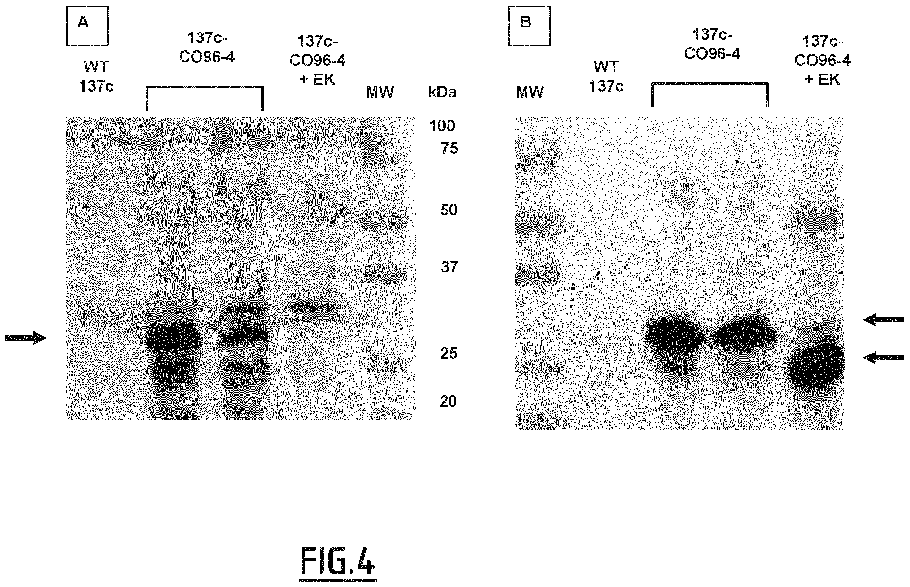

**Figure 4****:** Western Blot analysis of 137c-CO96-4 transformant using monoclonal anti-Flag M2 (A) or anti-HA (B) antibodies. 50 µg of total soluble protein from 137c-CO96 transformants extracted by sonication were digested (+EK; A: Lane 4; B: Lane 5) or not (A: Lane 2 and 3; B: 3 and 4) by enterokinase (EK). 50 µg of total soluble protein from wild type (WT) 137c extracted with SDS buffer lysis. MW: molecular weight standard. Arrows indicate the positions of recombinant proteins with or without the Flag Tag.

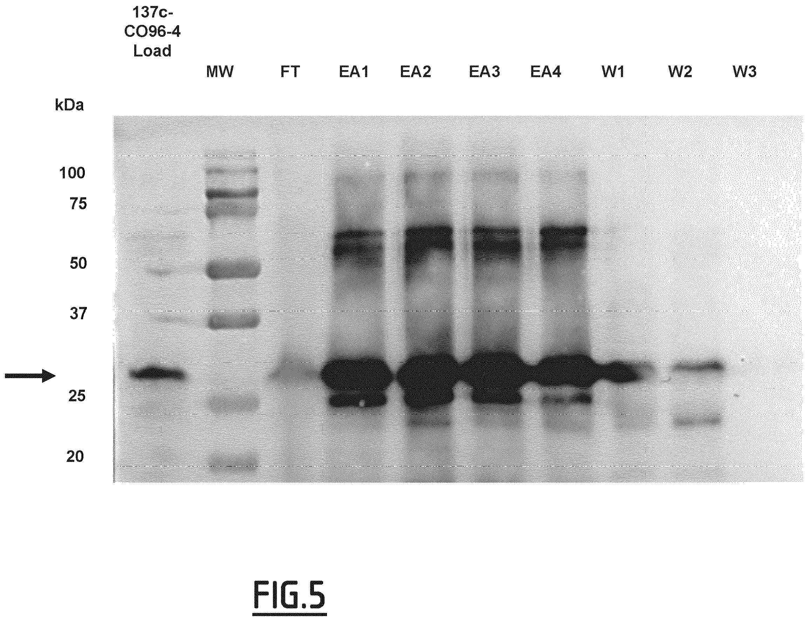

**Figure 5****:** Western Blot analysis of different elution fractions from anti-HA affinity chromatography performed on a protein extract from 137c-CO96-4 and using monoclonal anti-HA antibody. Protein samples (50 µg of protein extracted by sonication wild type (WT) 137c or 25 µl of elution or wash fractions) were loaded on a 15% SDS polyacrylamide gel. MW: molecular weight standard. Load: total soluble protein extracted by sonication before the incubation with anti-HA resin. FT: Flow through. EA: elution fraction. W: wash fraction. Arrows indicate the positions of purified recombinant proteins.

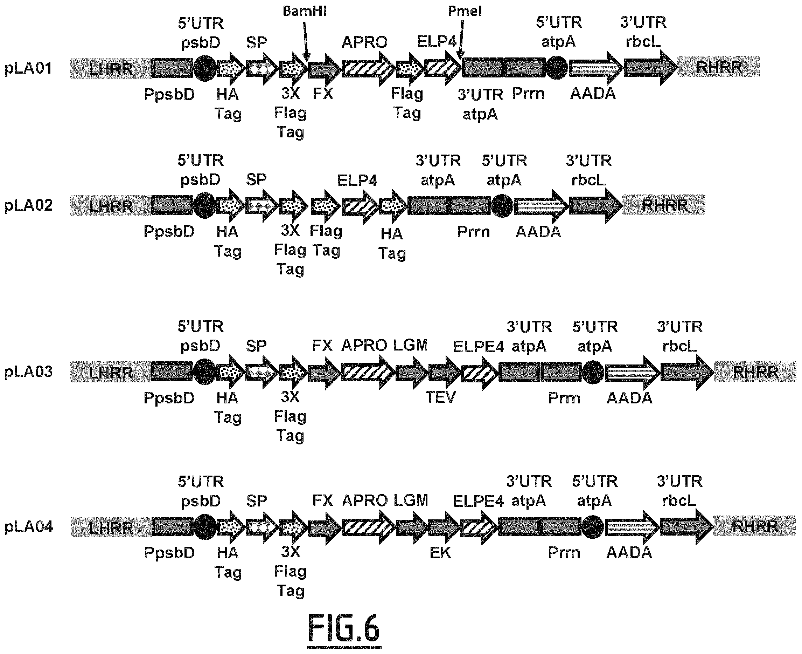

**Figure 6****:** Schematic presentation of the chloroplast transformation vectors for elastin polypeptide and peptide production.

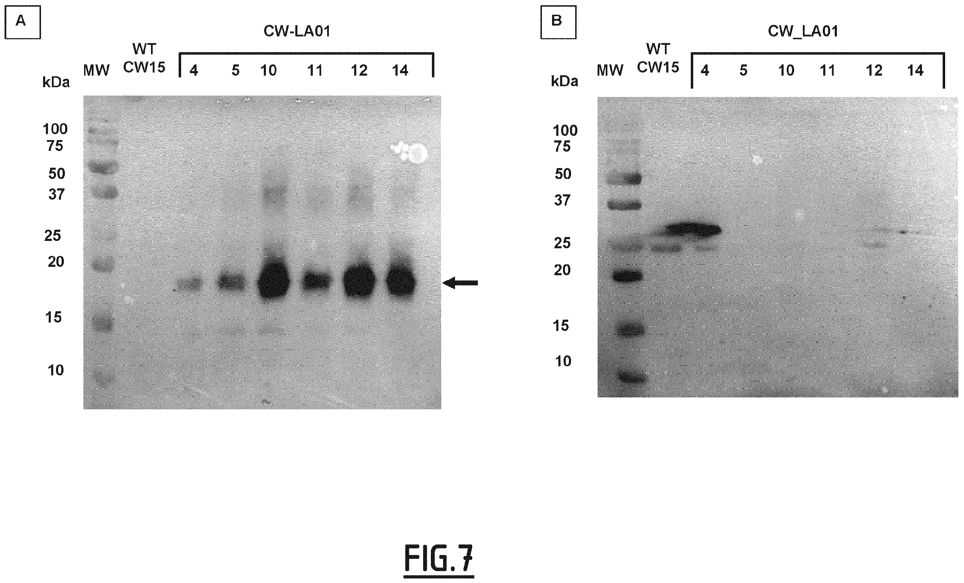

**Figure 7****:** Western blot analysis of algae cells transformed with pLA01, using monoclonal anti-Flag (A) or anti-HA (B) antibodies. 50 µg of total soluble protein samples extracted with SDS buffer lysis from wild type (WT) CW15 cells and from independent CW-LA01 transformants were separated on a 15% SDS polyacrylamide gel. MW: molecular weight standard. Arrows indicate the positions of recombinant proteins.

### EXAMPLES

### Example 1

### Material and Methods

All oligonucleotides and synthetic genes were purchased from Eurofins. All enzymes were purchased from NEB, Promega, Invitrogen and Sigma Aldrich/Merck. All plasmids were built on the pBluescript II backbone.

### Algal strains and growth conditions

The two algal strains used are the *Chlamydomonas reinhardtii* wild type (137c; mt+) and the cell wall deficient strain CW15 (CC-400; mt+), obtained from the *Chlamydomonas* Resource Center, University of Minnesota).

Prior to transformation, all strains were grown in TAP (Tris Acetate Phosphate) medium to mid-logarithmic phase (densities of approximately 1-2x10⁶ cell/mL) at a temperature comprised between 23°C to 25°C (ideally 25°C) on a rotary shaker in presence of constant light (70-150) µE/m²/s).

Transformants were grown in the same conditions and the same media supplemented with (containing) 100 µg/mL of spectinomycin or 100 µg/mL kanamycin, depending of the selectable marker gene present in the transformation vector.

Growth kinetics was also followed by measuring the optical density at 750 nm using a spectrophotometer.

### Algal transformation

*Chlamydomonas reinhardtii* cells were transformed using the helium gun bombardment technique of gold micro-projectiles complexed with transforming DNA, as described in the article Boynton *et al.*, 1988. Briefly, the *Chlamydomonas reinhardtii* cells were cultivated in TAP medium until midlog phase, harvested by gentle centrifugation, and then resuspended in TAP medium to a final concentration of 1.10⁸ cells/mL. 300 µL of this cell suspension was plated onto a TAP agar medium supplemented with 100 µg/mL of spectinomycin or 100 µg/mL of kanamycin, depending of the selectable marker gene present in the transformation vector. The plates were bombarded with gold particles (S550d; Seashell Technology) coated with transformation vector, as described by the manufacturer. The plates were then placed at 25°C under standard light conditions to allow selection and formation of transformed colonies.

### Total DNA extraction and PCR screening of positive transformants

Total DNA extraction was performed using the chelating resin Chelex 100 (Biorad) from single colonies (with size of around 1 mm in diameter) of wild type and/or antibiotic resistant transformants *Chlamydomonas* strains.

From isolated colonies, a quantity of cells corresponding to about 0.5 mm in diameter was removed with a pick and resuspended in 20 µL of H₂O. 200 µL of ethanol were added and incubated 1 min at room temperature. 200 µL of 5% Chelex were incorporated and vortexed. After an incubation of 8 min at 100 °C, the mixture was cooled down and centrifuged 5 min at 13,000 rpm. Finally, the supernatant was collected.

After transformation, algae colonies growing onto restrictive solid medium plates were expected to have the antibiotic resistant gene incorporated the transgene(s) into their genome.

In order to identify stable integration of the recombinant genes into the algal genome of the antibiotic resistant transformants were screened by Polymerase Chain Reaction (PCR or PCR amplification) in a thermocycler using 1 µL of total DNA previously extracted as template, two synthetic and specific oligonucleotides (primers) and Taq polymerase (GoTaq, Promega). The cycles of PCR amplification followed the guidelines recommended by the manufacturer. The PCR reactions were subjected to gel electrophoresis in order to check the PCR fragment of interest.

### Protein extraction, Western blot analyses

*Chlamydomonas* cells (50 mL, 1-2.10⁶ cells/mL) were collected by centrifugation. Cell pellet was resuspended in lysis buffer (50 mM Tris-HCI pH 6.8, 2% SDS, 10 mM EDTA). As further detailed below, in some embodiments of the example, the lysis buffer didn't contain 10 mM EDTA. After 30 min at room temperature, cell debris were removed by centrifugation at 13000 rpm and the supernatant containing the total soluble proteins was collected.

Depending on the further analysis step, total soluble proteins were extracted under non denaturing conditions in different buffers. Cell pellet was resuspended in a buffer containing 50 mM Tris-HCI (pH 6.8 or 8) or 20 mM Tris-HCI (pH 6.8 or 8). The sonication step was carried out with the algal cell suspension held on ice, using a cell disruptor a sonicator FB505 500W (Sonic/FisherBrand) and a micro-tip setting of 20 % power, with continuous sonication for 5 min. After sonication, cell debris were removed by centrifugation at 13000 rpm, 30 min.

Total soluble proteins present in the supernatant were quantified using the Pierce BCA protein assay kit, following the instructions of the supplier (Thermofisher).

Total soluble protein samples (50 or 100 µg or another quantity further mentioned in the example depending of the experiment) were separated in a 12 or 15% Tris-glycine SDS-PAGE prepared according to Laemmli (1970).

For experiments performed under reducing conditions, samples were prepared in Laemmli sample loading buffer with 50 mM DTT (or more depending of the fusion protein) or 5% Beta-mercaptoethanol, and further denaturated 5 min at 95°C before loading. The SDS PAGE experiments were carried out using a Protein gel tank from BioRad.

After separation, samples were blotted onto a nitrocellulose membrane (GE HealthCare) using standard transfer buffer and a Trans-Blot® Turbo™ Transfer System from Biorad. In order to visualize the transferred proteins, the nitrocellulose membrane were stained by Ponceau S dye. Membranes were further blocked with Tris-buffered saline Tween buffer (TBS-T) (50 mM Tris-HCI pH 7.5, 150 mM NaCl, 0.1% Tween-20) containing 5% Bovin Serum Albumin (BSA). After one hour of saturation at room temperature under gently shaking, membranes were incubated during one night at 4°C with TTBS buffer containing mouse primary antibody (See table 1).

**Table 1: primary antibodies**

| Primary antibody | Source | Dilution |
|---|---|---|
| Monoclonal ANTI-FLAG® M2 antibody produced in mouse | Sigma | 1:1000 |
| Monoclonal ANTI-HA PURIFIED antiobody produced in mouse IGG | Sigma | 1:6000 |
| Monoclonal ANTI-Aprotinin antibody produced in mouse | Abcam | 1:2000 or 1:3000 |

After three washes with TBS-T-BSA buffer, membranes were incubated one hour at room temperature with TBS-T-BSA buffer containing secondary antibodies (Promega). After four washes with TTBS buffer and one wash with TBS buffer, the membranes were revealed with a linked secondary antibody conjugated to horseradish peroxidase (HRP) (Anti-Mouse IgG (H+L), HRP Conjugate; Promega,) and visualized by enhanced chemiluminescence (ECL) using Clarity Max as ECL substrate (Biorad) and the ChemiDoc™ XRS+ system (Biorad).

### Protein purification

After centrifugation, algae cell pellets were resuspended in different buffers depending on the protein and on the further steps to which the protein is submitted. If the next step was an anti-FLAG M2 affinity chromatography, the buffer contained 50 mM Tris-HCI pH8, 500 mM NaCl and 0.1% Tween 20. If the next step was an anti-HA affinity chromatography, the buffer contained 20 mM Tris-HCI pH8. Approximately, 10 mL of buffer were used per g of wet algal cells, depending of the transformants. The resuspended cells were sonicated in the same conditions as previously described.

### Affinity chromatography

All recombinant proteins were tagged in their N-terminal with a Flag Tag epitope which will bind specifically on an anti-Flag M2 affinity gel (Sigma/Merck). This resin contains a mouse monoclonal Anti-Flag® M2 antibody that is covalently attached to agarose.

All steps of this experiment were carried out as described by the manufacturer. Briefly, the samples of total soluble proteins were filtered using a cellulose acetate 0.45 µm filter and mixed with anti-Flag® M2 affinity gel prepared as recommended by the manufacturer and equilibrated in binding buffer (50 mM Tris-HCl pH8, 500 mM NaCl, 0.1% Tween 20). Approximately, 1 mL of resin was used per 4 to 8 g of wet algal cells, depending of the transformants. Binding of the recombinant fusion protein was performed at 4°C for 4h or overnight with a gently and continuous end-over-end mixing. After incubation, the mixture of soluble protein incubated with resin were loaded by gravity on an empty Bio-rad Econo-pac column or collected by centrifugation, and washed several times with 40 column volumes of TBST and 20 column volumes of TBS. The protein of interest was eluted from the resin using 100 mM Glycine pH 3.5, 500 mM NaCl and neutralized with Tris-HCI pH 8 to a final concentration of 50 mM.

Some recombinant proteins were tagged with a HA epitope Tag which will bind specifically on an anti-HA agarose resin (Pierce/Thermo Scientific). All steps of this experiment were carried out as described by the manufacturer. Briefly, the filtered samples of total soluble proteins were mixed with an anti-HA agarose resin prepared as recommended by the manufacturer and equilibrated in TBS and incubated overnight at 4°C with a gently and continuous end-over-end mixing or a rocking platform. After incubation, the resin was pelleted 5 to 10 second at 12,000 g (repeat 3 times). The supernatant was kept for further analysis. The pelleted resin was washed several times with 10 bed volumes of TBST. The protein of interest was eluted from the resin after incubation 15 min at 30 °C of the resin with 10 bed volumes of 1 mg/ml Pierce HA peptide. The resin was pelleted by centrifugation (5 to 10 second at 12,000 g). The supernatant containing the fusion protein was collected. This elution step was repeat 3 additional times.

Each elution fractions of affinity chromatography were further analyzed by SDS-PAGE and Western Blot.

Depending of the further step, the elution fractions containing the protein of interest were dialyzed in Slide-A-Lyzer Dialysis Cassettes (3.5 kDa MWCO, Thermo Scientific) as described by the manufacturer against the buffer used in the further step, as for instance, for the protease digestion. The dialyzed samples were concentrated using Vivaspin 6 (3 kDa MWCO, GE Healthcare).

### Separation of the protein of interest from the carrier

The separation of the protein of interest from the carrier was made by protease digestion, in particular, in the present invention by enterokinase (light chain) or Tobacco Etch Virus (TEV) Protease from New England BioLabs (NEB).

Enzymatic digestions were performed as recommended by the manufacturer.

For example, for enterokinase light chain digestion, reactions combined 25 µg of protein of interest in 20 µL of buffer (20 mM Tris-HCl pH 8.0, 50 mM NaCl, 2 mM CaCl₂), with 1µL of enterokinase light chain. Incubation was made at 25°C for 16h.

For example, for TEV digestion, typical reaction recommended by the manufacturer combined 15 µg of protein substrate with 5 µL of TEV protease reaction buffer (10X) to make a 50 µL total reaction volume. After addition of 1 µL of TEV Protease, reaction was incubated at 30°C for 1 hour or 4°C overnight.

For example, for Factor Xa digestion, the manufacturer recommended to digest 50 µg of fusion protein with 1 µg of FXa in a volume of 50 µL at 23°C for 6h. The buffer reaction consisted in 20 mM Tris-HCl pH 8.0, 100 mM NaCl and 2 mM CaCl₂.

### Cleavage of the polypeptide by endoproteinase

The choice of the endoproteinase used to cleave the polypeptide of interest depends of the amino acid sequence of this polypeptide. Endoproteinases can be for instance, endoproteinase Glu-C, endoproteinase Arg-C, endoproteinase Asp-C, endoproteinase Asp-N, or endoproteinase Lys-C.

Enzymatic digestions were performed as recommended by the manufacturer. For example, for endoproteinase Glu-C digestion (from NEB), the manufacturer recommended to digest 1 µg of substrate protein with 50 ng of endoproteinase Glu-C at 37°C for 16h. The buffer reaction consisted in 50 mM Tris-HCl pH 8.0 and 0.5 mM GluC-GluC.

### Size exclusion chromatography (SEC)

Size-exclusion chromatography of purified and digested fusion protein was performed using an AKTA Pure system (GE Healthcare) in order to separate the protein of interest from the carrier.

A Superdex S30 Increase G10/300 GL column (GE Healthcare) and a HiLoad 26/600 Superdex 30 prep grade column were first calibrated using two standards diluted with 2X PBS buffer (or appropriate buffer for the further step): aprotinin (bovine lung; 6.5 kDa), and glycine (75 Da).

After a washing step in water, the Superdex S30 Increase G10/300 GL column was equilibrated in running buffer (2X PBS, pH 7.4, or 1X PBS, pH 7.4 or appropriate buffer for the further step) and 200 to 500 µL samples were run through the column at a rate of 0.5 mL/min. Elution of protein was detected by measuring optical absorbance at 280, 224 and 214 nm. 0.5 mL fractions were collected and analyzed by SDS-PAGE followed by Western-Blot or stained by Coomassie Blue dye.

After a washing step in water, the HiLoad 26/600 Superdex 30 prep grade column was equilibrated in running buffer (2X PBS, pH 7.4, or 1X PBS, pH 7.4 or the appropriate buffer for the further step) and samples (4 to 30 mL) were run through the column at a rate of 2.6 mL/min. Elution of proteins was detected by measuring optical absorbance at 280, 224 and 214 nm. 4 mL fractions were collected and analyzed by SDS-PAGE followed by Western-Blot.

In some embodiment, the elution fractions of interest were pooled and evaporated using a SpeedVac (Eppendorf). The peptides or polypeptides or proteins present in these evaporated samples were subjected to Edman degradation to confirm the amino acid sequence at the N-terminus of the protein of interest.

### Example 2

### Production of Collagen like protein, collagen like domain and collagen like polypeptide in the chloroplast of Chlamydomonas reinhardtii by chloroplast genome transformation

### Construction of transformation vector for the expression of the Collagen-Like Protein

In order to produce an innovating collagen-like protein (CLP) and/or collagen-like domain, we screened databases to find collagen-like genes encoding collagen-like domain from different origins. One of the sequence founded was the CCMP2712 protein from the microalgae *Guillardia theta* (*G. theta*)*.* The amino acid sequence of the CCMP2712 protein from *G. theta* (called GtCLP SEQ ID N°51) contained collagen like domain and was extracted from GenBank Accession Number XM_005827950.

Nothing was described about the capability of the protein encoded by the identified gene to form a collagen-like triple-helical structure.

In *Chlamydomonas reinhardtii,* the codon usage has been shown to play a significant role in protein accumulation (Franklin *et al*., 2002; Mayfield and Schultz, 2004).

The nucleic acid sequence encoding CCMP2712 protein was designed and optimized in order to improve its expression in *C. reinhardtii* host cell.
Methods for altering nucleic acid sequence for improved expression in host cell are known in the art, particularly in algae cell, particularly in *C. reinhardtii.*

A codon usage database was found at http://www.kazusa.or.jp/codon/. (See the codon usage for chloroplast genome of *C. reinhardtii*; Figure 1).

For improving expression in *C. reinhardtii* chloroplast of the gene of interest, codons from their native sequence which are not commonly used, were replaced with a codon coding for the same or a similar amino acid residue that is more commonly used in the *C. reinhardtii* chloroplast codon bias. In addition, other codons were replaced to avoid sequences of multiple or extended codon repeats, or some restriction enzyme site, or having a higher probability of secondary structure that could reduce or interfere with expression efficiency.

In order to check and to fulfill all criteria mentioned above, the amino acid sequence of the protein of interest were also optimized by the software GENEius of Eurofins using the appropriate codon usage for *C. reinhardtii* chloroplast.

After its codon optimisation, the gene *Gtclp* encoding the native CCMP2712 protein from *G. theta* (called "recombinant GtCLP" or 3F-TV-GtCLP-TV-HA) was designed to be operationally fused at its 5'end to the codon optimized nucleic acid sequences encoding an amino acid sequence containing the 3xFlag epitope Tag (SEQ. DYKDDDDKDYKDDDDKDYKDDDDK; SEQ ID N°25) followed by the recognition site of the TEV protease (SEQ ENLYFQG; SEQ ID N°52). At its 3'end, the optimized gene *Gtclp* were operationally fused to the optimized nucleic acid sequence coding for the recognition site of the TEV protease followed by the HA epitope Tag (SEQ ID N°26). The recombinant GtCLP produced *in vivo* in *C. reinhardtii* chloroplast was also called 3F-TV-GtCLP-TV-HA. The two recognition sites of the TEV protease allowed the elimination of the 3xFlag and HA Tags by *in vitro* protease digestions.

This resulting fusion gene *3f-tv-Gtclp-tv-ha* (SEQ ID N°10) encoding the recombinant GtCLP called 3F-TV-GtCLP-TV-HA (SEQ ID N°55) was synthesized and cloned by Eurofins Genomics into the vector pEX-A258 resulting in vector pAL70.

After PCR amplification from the vector pAL70 using the primers O5'SCL70 (SEQ ID N°56) and O3'SCL70 (SEQ ID N°57), the PCR fragment FPCR-SCL70 of 1317 bp (SEQ ID N°58) was cloned using the Gibson Assembly of New England Biolabs (as recommended by the manufacturer) into the expression cassette of the gene of interest (*goi*) present in the chloroplast transformation vector pLE56 linearized by *Nco*I and *Sal*I to form the vector pCO86 (Figure 2).

The chloroplast expression vector pLE56 contained two expression cassettes for the expression of the genes encoding the selectable marker (*gos*) and the recombinant protein of interest (*goi*)*.* The selection cassette contained the selectable marker *aadA* gene coding aminoglycoside 3"-adenylyltransferase and conferring the resistance to spectinomycin and streptomycin. This gene was operationally linked at its 5' end to the *C. reinhardtii 16S rRNA* promoter (*Prrn*) fused to the *atpA* 5'UTR and at its 3'end to the 3'UTR of the *C. reinhardtii rbcL gene.* In the second cassette, stable expression of the recombinant *goi* was controlled by the promoter and 5'UTR from *C. reinhardtii psbD* and the 3'UTR from *C. reinhardtii atpA.*

These two expression cassettes were flanked by a left (LHRR) and right (RHRR) endogenous homologous recombination sequences which were identical to those surrounding the targeted integration site into the *C. reinhardtii* chloroplast genome. The choice of the insertion site within the chloroplast genome was generally made such as not to disrupt an essential gene or interrupt the expression of a polycistronic unit. In a preferred embodiment, the chloroplast transformation vectors in the present invention allowed the targeted integration of the transgenes into the chloroplast genome of *C. reinhardtii* between the *5S rDNA* and *psbA* genes (and derives from instance from the sequence GenBank Accession Number NC005352).

### Construction of chloroplast transformation vector for the expression of the chimeric Collagen-Like domain of G. theta CCMP2712 CLP.

The collagen-like domain from *G. theta* CCMP2712 protein (SEQ ID N°59; named GtCLD) was designed in order to fuse at its N-terminus with an amino acid sequence containing a Cys Knot and CR4 Repeat sequences (SEQ. GPCCGPPGPPGPPGPP, SEQ ID N°60). The Cys Knot and CR4 Repeat sequences were known in the art as adding conformational rigidity of sequence. At its C-terminus, GtCLD was fused with a CR4 Repeat followed by Cys Knot sequences and the Foldon fibritin of the T4 phage (SEQ. GYIPEAPRDGQAYVRKDGEWVLLSTFL, SEQ ID N°61). The resulting recombinant protein was named chimeric collagen-like domain from *G. theta* or GtCCLD (SEQ ID N°62). The GtCCLD protein was also designed to be fused at its C-terminus a 3xHA epitope Tag (called 3HA; SEQ ID N°63: YPYDVPDYAYPYDVPDYAYPYDVPDYA).

The synthetic codon optimized gene *Gtccld-3ha* (SEQ ID N°84) were synthetized and cloned in the vector pEX-A258 by Eurofins Genomics to form the pAL81. The gene *Gtccld-3ha* was subcloned in the *goi* expression cassette of the chloroplast transformation vector pLE63 previously linearized by *BamH*I and *Pme*I digestions to give pCO96. More precisely, *Gtccld-3ha* was subcloned downstream the nucleic acid sequence *ha-sp-3F* encoding the HA epitope Tag (HA) linked to the signal peptide (SP) followed by the 3xFlag epitope Tag (HA-SP-3F) (Figure 2).

Then, in *C. reinhardtii* chloroplast, the recombinant GtCCLD-3HA (SEQ ID N°64) was produced fused at its N-terminus with the amino acid sequence HA-SP-3F and was called HA-SP-3F-GtCCLD-3HA (SEQ ID N°83). This recombinant protein was encoded by the nucleic acid sequence called *ha-sp-3f-Gtccld-3ha* (SEQ ID N°19). The chloroplast expression vector pLE63 contained the same expression cassette for the selectable marker as pLE56 (Figure 2). Stable expression of the *goi* was controlled by the promoter and 5'UTR from *C. reinhardtii psbD* and the 3'UTR from *C. reinhardtii atpA.* In this expression cassette, the *goi* was fused at its 5'end to a nucleic acid sequence *ha-sp-3F.* pLE63 allowed the same targeted integration of the recombinant genes into the *C*. *reinhardtii* chloroplast genome as pLE56 and pCO86.

In order to remove the nucleic sequence encoding the 3xHA Tag, the PCR fragment *Gtccld-2* (SEQ ID N°65) containing the nucleic sequence encoding the recombinant chimeric collagen-like domain from *G. theta* GtCCLD was amplified by PCR from pAL81 and cloned by Gibson assembly between the *psbD* promoter/5'UTR and the *atpA* 3'UTR into pLE63 linearized by *BamH*I and *Pme*I to form pCO26 (Figure 2). As in pCO96, the gene *ccld-2* was subcloned downstream the nucleic acid sequence *ha-sp-3F* encoding the amino acid sequence HA-SP-3F (Figure 2).

Then, in *C. reinhardtii* chloroplast, the recombinant chimeric collagen-like domain from *G. theta* GtCCLD was produced fused at its N-terminus with the amino acid sequence HA-SP-3F and was called HA-SP-3F-GtCCLD (SEQ ID N°85) encoded by the nucleic acid sequence *ha-sp-3f-Gtccld* (SEQ ID N°20).

In order to produce only the collagen-like domain from *G. theta* without Cys Knot and CR4 Repeat sequences, the PCR fragment *Gtcld* (SEQ ID N°66) containing the nucleic sequence the gene was amplified by PCR from pAL81 and cloned by Gibson assembly between the *psbD* promoter/5'UTR and the *atpA* 3'UTR into pLE63 linearized by *BamH*I and *Pme*I to form pCO28 (Figure 2). As in pCO96 and pCO26, the gene *Gtcld* was subcloned downstream the nucleic acid sequence *ha-sp-3F* encoding the amino acid sequence HA-SP-3F (Figure 2).

Then, in *C. reinhardtii* chloroplast, the recombinant collagen-like domain from *G*. *theta* GtCLD was produced fused at its N-terminus with the amino acid sequence HA-SP-3F and was called HA-SP-3F-GtCLD (SEQ ID N°86) encoded by the nucleic acid sequence *ha-sp-3f-Gtcld* (SEQ ID N°21).

After their production in algae chloroplast, the signal peptide (SP) will be cleaved *in vivo* from the recombinant proteins HA-SP-3F-GtCCLD-3HA, HA-SP-3F-GtCCLD and HA-SP-3F-GtCLD during their translocations into the thylakoids. Thus, 3 others proteins could be produced 3F-GtCCLD-3HA, 3F-GtCCLD and 3F-GtCLD.

The 3xFlag Tag will be cleaved by *in vitro* enterokinase digestion of these recombinant proteins.

### Transformation of algae

The transformation vectors pCO86, pCO96, pCO26 and pCO28 were bombarded in *C. reinhardtii* cells (137c and CW15) as described in the example 1.

In order to identify stable integration of the recombinant genes encoding fusion protein into the chloroplast algal genome, spectinomycin resistant colonies were screened by PCR analysis. For positive PCR screens of the fusion protein gene in CO96, CO26 and C028 transformants, the primers O5'ASTatpA2 (SEQ ID N°67) and O3'SUTRpsbD (SEQ ID N°68) annealing, respectively, in the *atpA* 3'UTR and *psbD* 5'UTR were used. For CO86 transformants, two other primers were used O5'SCL70 (SEQ ID N°69) and O5'PpsbDCla2 (SEQ ID N°70), annealing, respectively, in the gene of interest and *psbD* promoter.

### Analyses and results

Western Blot analysis were performed in reducing conditions on total soluble protein samples extracted from several transformants obtained after transformation with pCO86, pCO96, pCO26 and pCO28 (from 137c and CW15 strains).

The results showed that the four recombinant proteins were produced, being probed by the anti-Flag antibody and/or anti-HA antibody.

The Figure 2 showed the results of Western Blots on total soluble protein extracts from CW-CO86 and CW-CO96 transformants producing the recombinant proteins, 3F-TV-GtCLP-TV-HA and HA-SP-3F-GtCCLD-3HA.

In order to release the recombinant proteins of interest from the N-terminus epitope Tag fused or not with the signal peptide SP, total soluble protein samples extracted from one clone of CO96, CO26 and CO28 transformants were digested *in vitro* by enterokinase digestion as described in example 1. The Figure 4 showed the western blots performed on the enterokinase digestion of total soluble protein samples extracted from 137c-CO96-4 transformant. The recombinant protein HA-SP-3F-GtCCLD-3HA produced *in vivo* was cleaved to from GtCCLD-3HA.

In the case of CO86 transformants producing the recombinant protein 3F-TV-GtCLP-TV-HA, total soluble protein samples were digested *in vitro* either by enterokinase to give the protein TV-GtCLP-TV-HA or by TEV protease to give the protein GtCLP.

137c-CO96-4 cells were produced from around 900 mL cultures. Algae cells (around 3 g) were resuspended and sonicated as described in the example 1. 14.6 mL of total soluble protein extract was obtained.

Recombinant protein from 6.5 mL protein extract from 137c-CO96-4 were purified by affinity chromatography using anti-HA resin (150 µl). Elution fractions were analysed by Western Blot analysis. As an example, the results, shown in Figure 5 revealed the effectiveness of the affinity chromatography purification of the chimeric collagen like domain.

Western Blot analysis performed in non-reducing conditions (without DDT and boiling or not the protein sample before loading onto the polyacrylamide gel) showed that the chimeric collagen like domain produced in CO96 transformants formed *in vivo* multimeric structures of high apparent molecular weight in contrast to an apparent molecular weight of the protein in reducing conditions.

### Example 3

### Production of elastin like peptides or polypeptides or derivatives in a fusion protein using aprotinin as a carrier in the chloroplast of Chlamydomonas reinhardtii by chloroplast genome transformation

### Construction of transformation vectors (pLA01, pLA02, pAL03 and pAL04)

In chloroplast transformation vector, ELP4, an elastin like polypeptide consisted of a repeat of the VGVAPG hexapeptide (SEQ ID N°5), more particularly of a 4-fold repeat of this hexapeptide (SEQ ID N°81: VGVAPGVGVAPGVGVAPGVGVAPG), was expressed in a fusion protein in which it was fused, at the C-terminus of the chimeric aprotinin HA-SP-3F-FX-APRO (SEQ ID N°41 and 42). This fusion partner contained aprotinin fused at its N-terminus to an amino acid sequence made of the HA epitope Tag (HA) followed by the signal peptide (SP), the 3xFlag epitope Tag (3F), and the cleavage site for Factor Xa (FX; SEQ ID N°71: IEGR). The Flag epitope Tag sequence (SEQ ID N°24: DYKDDDDK) which is the cleavage site for the enterokinase was inserted between the chimeric aprotinin and ELP4 in order to allow the release of ELP4 from aprotinin by *in vitro* site specific proteolysis of the fusion protein with enterokinase.

After the production in algae chloroplasts of the fusion protein HA-SP-3F-FX-APRO-F-ELP4 (SEQ ID N°87), the N-terminus fragment HA-SP will be cleaved during protein translocation into the thylakoids, and the following recombinant protein 3F-FX-APRO-F-ELP4 will be produced *in vivo.*

As explained in Example 2, in *Chlamydomonas reinhardtii*, the codon usage in the nucleic acid sequence encoding protein of interest has been shown to play a significant role in protein accumulation.

The nucleic acid sequence encoding the aprotinin were designed and optimized as described in Example 2 in order to improve their expression in *C. reinhardtii* host cells. After optimization, the gene encoding aprotinin (APRO) were operationally fused at its 5'end to a codon optimized nucleic acid sequences encoding the HA epitope Tag (HA) followed by a signal peptide, the 3xFlag epitope Tag (3F) and the cleavage site recognized by the Factor Xa protease (FX) to form the chimeric aprotinin gene *ha-sp-3f-fx-apro* (SEQ ID N°42).

The nucleic acid sequence encoding ELP4 was first codon-optimized using also the same method described in Example 1 and the codon usage for chloroplast genome of *Chlamydomonas.* The resulting sequence was used to design two overlapping oligomers O5'Gibs-ELP4 (SEQ ID N°72) and Q3'Gibs-ELP4 (SEQ ID N°73) which were used as primers and template to amplify by PCR the fragment FGibs-ELP4 of 194 bp. This amplified DNA were cloned using the Gibson Assembly Master Mix from New England Biolabs (as recommended by the manufacturer) into the chloroplast transformation vector pAU76 and linearized by *Pme*I to form the vector pLA00.

The expression vector pAU76 for chloroplast genome transformation contained two expression cassettes for the expression of the genes encoding the selectable marker (identical to previous transformation vector) and the chimeric aprotinin HA-SP-3F-FX-APRO. pAU76 allowed the same targeted integration of the recombinant genes into the *C*. *reinhardtii* chloroplast genome as pLE63.

The nucleic acid sequence encoding ELP4 were amplified by PCR from pLA00 using the primers O5'Gibs01BE (SEQ ID N°74) and O3'Gibs01BE (SEQ ID N°75). The PCR fragment FPCR-AP-FELP4 (SEQ ID N°76) of 359 pb were cloned using the Gibson Assembly Master Mix into the pLE63 linearized by *BamH*I and *Pme*I to form the vector pLA01. The transformation vector pLA01 allowed the production of the fusion protein HA-SP-3F-FX-APRO-F-ELP4 containing ELP4 linked at its N-terminus to the chimeric aprotinin HA-SP-3F-FX-APRO followed by the 1X Flag Tag (Figure 6).

The chloroplast transformation vector pLA02 was obtained by cloning by Gibson Assembly into pLE63 (linearized by *BamH*I and *Pme*I), the PCR fragment FPCR-FELP4-HA (SEQ ID N°77) (359 pb) amplified from pLA00 with primers O5'Gibs02BE (SEQ ID N°78) and O3'Gibs02BE (SEQ ID N°79). The transformation vector pLA02 allowed the production of fusion protein HA-SP-3F-1F-ELP4 containing ELP4 linked at its N-terminus to the chimeric sequence HA-SP-3F followed by the 1X Flag Tag (Figure 6).

In the case of algae chloroplasts transformed by pLA01 or pLA02 and if the signal peptide SP is cleaved after translocation of the fusion protein into the lumen of thylakoids, two other different proteins can be produced *in vivo,* 3F-FX-APRO-1F-ELP4 or 3F-1F-ELP4 (SEQ ID N°88).

In both types of transformation vectors, the release of ELP4 from the fusion proteins can be performed *in vitro* by enterokinase digestion which cleaved the protein sequence after the second lysine amino acid in the motif sequence DYKDDDDK (SEQ ID N°24) present in the 1X Flag Tag just upstream ELP4.

The elastin like polypeptide named ELPE4 consisted of a repeat of the VGVAPGE (SEQ ID N°9), a derivative of the peptide VGVAPG, more particularly of a 4-fold repeat of this peptide (SEQ ID N°80, VGVAPGEVGVAPGEVGVAPGEVGVAPGE). In chloroplast transformation vector, ELPE4 was also expressed in a fusion protein in which it was fused at the C-terminus of the chimeric aprotinin HA-SP-3F-FX-APRO.

In order to separate *in vitro* by the ELPE4 from the carrier, a flexible linker LGM (SEQ ID N°50: RSGGGGSSGGGGGGSSRS) followed by a cleavage site for TEV protease (TV; SEQ ID N°52: ENLYFQG) or enterokinase (EK; SEQ ID N°38: DDDDK) were added.

Two types of fusion proteins have been produced from two different chloroplast expression vectors: HA-SP-3F-FX-APRO-LGM-TV-ELPE4 (SEQ ID N°89) or HA-SP-3F-FX-APRO-LGM-EK-ELPE4 (SEQ ID N°90).

The nucleic acid sequence encoding LGM-TV-ELPE4 or LGM-EK-ELPE4 were codon-optimized using also the same method described in Example 1 and the codon usage for chloroplast genome of *C. reinhardtii.* After codon optimization, the different synthetic genes *Igm-tv-elpe4* (SEQ ID N°40) and *Igm-ek-elpe4* (SEQ ID N°48) were synthetized by Eurofins. These optimized genes were cloned by Gibson assembly downstream the gene encoding the carrier into an expression cassette (SEQ N°82) present in the chloroplast transformation vector pAU76 linearized by *Pme*I to give respectively, pLA03 and pLA04.

### Transformation of algae

The transformation vectors pAL01, pLA02, pLA03 and pLA04 were bombarded in *C. reinhardtii* cell (137c and CW15) as described in the Example 1.

In order to identify stable integration of the recombinant genes encoding fusion protein into the chloroplast algal genome, spectinomycin resistant colonies were screened by PCR analysis using the primers O5'ASTatpA2 (SEQ ID N°67) and O3'SUTRpsbD (SEQ ID N°54) annealing, respectively, in the *atpA* 3'UTR and *psbD* 5'UTR.

### Analyses and results

Western Blot analysis performed using anti-Flag antibody on total soluble proteins extracted from different independent strains of LA01, LA03 or LA04 transformants revealed that the fusion proteins HA-SP-3F-FX-APRO-F-ELP4 (SEQ ID N°87), HA-SP-3F-FX-APRO-LGM-TV-ELPE4 (SEQ ID N°89) and HA-SP-3F-FX-APRO-LGM-EK-ELPE4 (SEQ ID N°91) were produced in the *C. reinhardtii* chloroplast.

As shown in the Figure 7, Western Blot analysis showed that the ELP4 polypeptide fused to 3F-FX-APRO is very well produced in the LA01 transformants using anti-Flag antibody. Moreover, in all LA01 transformants, the HA epitope Tag and the signal peptide seems to be cleaved because Western blots performed on the same total soluble protein extracts showed that the primary anti-HA antibody didn't recognize any fusion protein (Figure 7).

In the LA02 transformants, no recombinant protein were detected (Figure 7) demonstrating that the fusion of ELP4 to 3F-FX-APRO (or aprotinin as a general manner) allows the accumulation of ELP4.

Biomass of one transformant CW-LA01 was produced. Cell pellet was resuspended in sonication buffer.

Fusion protein were purified by anti-Flag M2 affinity chromatography. Elution fraction containing the fusion protein were identify by Western Blot analysis, dialyzed and concentrated. Enterokinase protease digestions were performed followed by a size exclusion chromatography (HiLoad 26/00 Superdex 30) allowing the purification of the polypeptide ELP4.

The same method was applied for the purification of the ELPE4. The fusion protein were purified by affinity chromatography. Elution fraction containing the fusion protein were identify by Western Blot analysis, dialyzed and concentrated. Enterokinase or TEV protease digestions were performed depending on the transformant followed by a size exclusion chromatography (HiLoad 26/00 Superdex 30) allowing the purification of the polypeptide ELPE4.

In the case of LA03 transformants and after the TEV protease digestion, the released polypeptide was GVGVAPGEVGVAPGEVGVAPGEVGVAPGE (SEQ ID N°53).

In order to cleave by endoproteinase the polypeptides ELPE4 into peptides VGVAPGE, the SEC elution fractions were evaporated and dialyzed for salts removing and buffer changing, using a dialysis tube with a 1 kDa cutoff.

After digestion by the Glu-C_endoproteinase of the dialyzed samples as described in the Example 1, the released peptides were purified by a size exclusion chromatography.

## Claims

1. A recombinant microalgae comprising a nucleic acid sequence encoding a recombinant protein, polypeptide or peptide comprising repeat units of amino acids, said protein, polypeptide or peptide being chosen from collagen, elastin and their derivatives, and said nucleic acid sequence being located in the chloroplast genome of microalgae.

2. A method for producing a recombinant protein, polypeptide or peptide comprising repeat units of amino acids in the chloroplast of microalgae, said protein, polypeptide or peptide being chosen from collagen, elastin and their derivatives, wherein said method comprises transformation of the chloroplast genome of microalgae with a nucleic acid sequence encoding said recombinant protein, polypeptide or peptide.

3. Method according to claim 2, comprising:
(i) providing a nucleic acid sequence encoding said recombinant protein, polypeptide or peptide;
(ii) introducing the nucleic acid sequence according to (i) into an expression vector which is capable of expressing the nucleic acid sequence; and
(iii) transforming the chloroplast genome of microalgae host cell by the expression vector.

4. Method according to claim 3, further comprising:
(iv) identifying the transformed microalgae host cell;
(v) characterizing the microalgae host cell for the production of recombinant protein, polypeptide or peptide expressed from said nucleic acidssequence;
(vi) extracting the recombinant protein, polypeptide or peptide; and optionally
(vii) purifying the recombinant protein, polypeptide or peptide.

5. Method according to any claims 3 or 4, wherein said expression vector also comprises at least one expression cassette, said at least one expression cassette comprising the nucleic acid sequence encoding said recombinant protein, polypeptide or peptide.

6. Recombinant microalgae according to claim 1 or method according to any of claims 2 to 5, wherein the nucleic acid sequence encoding the protein, polypeptide or peptide is codon optimized for expression in the chloroplast genome of the microalgae host cell.

7. Recombinant microalgae according to claim 1 or 6 or method according to any of claims 2 to 6, wherein said protein, polypeptide or peptide derivative consists in an amino acid sequence at least 80% identical to the amino acid sequence of the recombinant peptide, polypeptide or protein.

8. Recombinant microalgae according to any of claims 1 or 6 to 7 or method according to any of claim 3 to 7, wherein said nucleic acid sequence encoding a recombinant protein, polypeptide or peptide is fused operationally at its 5' or 3'end to a nucleic acid sequence encoding a carrier.

9. Recombinant microalgae according to any of claims 1 or 6 to 8 or method according to any of claim 3 to 8, wherein said nucleic acid sequence encoding a recombinant protein, polypeptide or peptide is operably linked to at least one regulatory sequence chosen from the *psbD* promoter and 5'UTR or the *16S rRNA* promoter *(Prrn)* promoter fused with the *atpA* 5'UTR, the *psaA* promoter and 5'UTR, the *atpA* promoter and 5' UTR and the *atpA* and *rbcL* 3'UTRs.

10. Method according to any claims 5 to 9, wherein said at least one expression cassette further comprises a nucleic acid sequence encoding an epitope Tag peptide fused operationally at its 5' or 3'end to the said nucleic acid sequence encoding the recombinant protein, polypeptide or peptide.

11. Method according to any of claims 5 to 10, wherein said at least one expression cassette further comprises a nucleic acid sequence encoding a signal peptide.

12. Method according to any of claims 5 to 11, wherein said at least one expression cassette further comprises a nucleic acid sequences encoding an amino acid sequence allowing the production of the said recombinant protein, polypeptide or peptide in specific cell compartment.

13. Recombinant microalgae according to any of claims 1 and 6 to 9 or method according to any of the claims 2 to 12, wherein said microalgae is chosen from the group consisting of *Chlorophyta, Chlorophyceae, Pleurastrophyceae, Prasinophyceae, Chromophyta, Bacillariophyceae* (diatoms), *Chrysophyceae, Phaeophyceae, Eustigmatophyceae, Haptophyceae, Raphidophyceae, Xanthophyceae, Cryptophyta, Cryptophyceae, Rhodophyta, Porphyridiophycea, Stramenopiles, Glaucophyta, Glaucocystophyceae, Chlorarachniophyceae, Haptophyceae, Dinophyceae, Scenedesmaceae, Euglenophyta, Euglenophyceae.*

14. Recombinant microalgae or method according to claim 13, wherein said microalgae is chosen from the group consisting of *Chlamydomonas, Chlorella, Dunaliella, Haematococcus,* diatoms, *Scenedesmaceae, Tetraselmis, Ostreococcus, Porphyridium,* and *Nannochloropsis.*

15. Use of a recombinant microalgae according to anyone of claims 1, 6-9, or 13-14, for producing a recombinant protein, polypeptide or peptide comprising repeat units of amino acids, said protein, polypeptide or peptide being chosen from collagen, elastin and their derivatives.


SEQUENCE LISTING

<110>  Alganelle

<120>  Recombinant microalgae able to produce peptides, polypeptides or
       proteins of collagen, elastin and their derivatives in the
       chloroplast of microalgae and associated method thereof

<130>  BET19L4540

<160>  91

<170>  PatentIn version 3.5

<210>  1
<211>  5
<212>  PRT
<213>  Artificial Sequence

<220>
<223>  elastin pentapeptide

<400>  1

Lys Gly Gly Val Gly
1               5


<210>  2
<211>  5
<212>  PRT
<213>  Artificial Sequence

<220>
<223>  Elastin pentapeptide

<400>  2

Val Gly Gly Val Gly
1               5


<210>  3
<211>  5
<212>  PRT
<213>  Artificial Sequence

<220>
<223>  Elastin pentapeptide

<400>  3

Gly Val Gly Val Pro
1               5


<210>  4
<211>  5
<212>  PRT
<213>  Artificial Sequence

<220>
<223>  Elastin pentapeptide

<220>
<221>  MISC_FEATURE
<222>  (4)..(4)
<223>  X is V, I or K

<400>  4

Val Pro Gly Xaa Gly
1               5


<210>  5
<211>  6
<212>  PRT
<213>  Artificial Sequence

<220>
<223>  Elastin hexapeptide

<400>  5

Val Gly Val Ala Pro Gly
1               5


<210>  6
<211>  7
<212>  PRT
<213>  Artificial Sequence

<220>
<223>  Elastin heptapeptide

<400>  6

Leu Gly Ala Gly Gly Ala Gly
1               5


<210>  7
<211>  9
<212>  PRT
<213>  Artificial Sequence

<220>
<223>  Elastin nonapeptide

<400>  7

Leu Gly Ala Gly Gly Ala Gly Val Leu
1               5


<210>  8
<211>  7
<212>  PRT
<213>  Artificial Sequence

<220>
<223>  Elastin derivative

<400>  8

Val Gly Val Ala Pro Gly Asp
1               5


<210>  9
<211>  7
<212>  PRT
<213>  Artificial Sequence

<220>
<223>  Elastin derivative

<400>  9

Val Gly Val Ala Pro Gly Glu
1               5


<210>  10
<211>  1251
<212>  PRT
<213>  Artificial Sequence

<220>
<223>  3f-tv-Gtclp-tv-ha

<400>  10

Ala Thr Gly Gly Cys Ala Gly Ala Thr Thr Ala Thr Ala Ala Ala Gly
1               5                   10                  15

Ala Cys Gly Ala Thr Gly Ala Thr Gly Ala Thr Ala Ala Ala Gly Ala
            20                  25                  30

Thr Thr Ala Cys Ala Ala Ala Gly Ala Cys Gly Ala Thr Gly Ala Cys
        35                  40                  45

Gly Ala Cys Ala Ala Ala Gly Ala Cys Thr Ala Thr Ala Ala Ala Gly
    50                  55                  60

Ala Thr Gly Ala Thr Gly Ala Cys Gly Ala Thr Ala Ala Ala Gly Ala
65                  70                  75                  80

Ala Ala Ala Cys Cys Thr Thr Thr Ala Thr Thr Thr Thr Cys Ala Ala
                85                  90                  95

Gly Gly Thr Ala Gly Thr Cys Gly Thr Cys Gly Thr Cys Gly Thr Cys
            100                 105                 110

Ala Ala Gly Gly Thr Gly Cys Thr Gly Cys Thr Gly Cys Cys Gly Cys
        115                 120                 125

Ala Gly Cys Cys Gly Cys Thr Gly Gly Thr Gly Cys Ala Gly Cys Ala

130                 135                 140

Gly Gly Ala Thr Thr Ala Ala Thr Cys Thr Thr Ala Cys Thr Thr Gly
145                 150                 155                 160

Thr Gly Thr Thr Ala Cys Thr Thr Thr Cys Ala Thr Thr Ala Gly Thr
                165                 170                 175

Ala Thr Cys Thr Thr Thr Ala Cys Gly Thr Gly Cys Thr Cys Ala Thr
            180                 185                 190

Gly Ala Ala Cys Gly Cys Ala Ala Ala Cys Cys Ala Ala Cys Ala Thr
        195                 200                 205

Cys Thr Thr Thr Ala Gly Thr Thr Gly Ala Ala Gly Gly Thr Thr Cys
    210                 215                 220

Ala Cys Gly Cys Ala Cys Thr Ala Cys Ala Ala Thr Gly Ala Thr Gly
225                 230                 235                 240

Cys Cys Ala Ala Cys Ala Thr Gly Gly Ala Ala Ala Gly Thr Ala Ala
                245                 250                 255

Cys Thr Gly Gly Thr Ala Cys Ala Thr Thr Ala Ala Ala Thr Cys Cys
            260                 265                 270

Ala Gly Thr Thr Gly Ala Ala Gly Ala Ala Ala Cys Ala Gly Ala Gly
        275                 280                 285

Ala Ala Ala Gly Ala Ala Gly Thr Ala Gly Ala Ala Gly Ala Ala Gly
    290                 295                 300

Ala Ala Cys Cys Ala Thr Cys Thr Thr Cys Ala Cys Cys Thr Cys Cys
305                 310                 315                 320

Ala Cys Cys Ala Cys Cys Ala Gly Cys Ala Gly Thr Ala Cys Ala Ala
                325                 330                 335

Gly Cys Thr Ala Cys Ala Cys Cys Ala Gly Ala Thr Gly Thr Ala Cys
            340                 345                 350

Cys Thr Cys Ala Ala Gly Ala Gly Cys Ala Ala Gly Ala Ala Gly Cys
        355                 360                 365

Cys Gly Ala Ala Cys Cys Ala Ala Gly Thr Cys Ala Gly Thr Cys Ala
    370                 375                 380

Gly Ala Ala Thr Gly Gly Gly Thr Thr Cys Cys Ala Cys Cys Ala Cys
385                 390                 395                 400

Cys Thr Ala Ala Ala Cys Cys Thr Thr Gly Gly Gly Ala Ala Cys Cys
                405                 410                 415

Ala Thr Thr Ala Gly Cys Thr Gly Ala Ala Ala Ala Ala Ala Ala Thr
            420                 425                 430

Ala Thr Thr Gly Gly Thr Cys Gly Thr Thr Thr Ala Gly Ala Ala Cys
        435                 440                 445

Gly Thr Ala Cys Thr Ala Ala Thr Cys Gly Thr Gly Ala Ala Gly Thr
    450                 455                 460

Ala Gly Thr Thr Cys Gly Thr Gly Gly Thr Gly Ala Thr Ala Thr Gly
465                 470                 475                 480

Gly Ala Ala Ala Ala Thr Gly Cys Ala Ala Ala Cys Ala Thr Gly Ala
                485                 490                 495

Thr Ala Ala Ala Ala Ala Ala Ala Thr Thr Ala Cys Gly Thr Gly Cys
            500                 505                 510

Thr Gly Cys Thr Ala Thr Gly Cys Gly Thr Thr Thr Ala Thr Thr Ala
        515                 520                 525

Ala Ala Gly Ala Ala Ala Cys Ala Ala Thr Thr Thr Gly Cys Ala Gly
    530                 535                 540

Cys Thr Ala Ala Ala Ala Thr Thr Gly Thr Gly Ala Ala Thr Thr Thr
545                 550                 555                 560

Ala Gly Ala Ala Cys Ala Ala Gly Cys Thr Thr Ala Thr Gly Ala Thr
                565                 570                 575

Cys Gly Thr Cys Gly Thr Thr Thr Ala Gly Cys Thr Cys Gly Thr Gly
            580                 585                 590

Ala Ala Ala Gly Thr Cys Gly Thr Ala Thr Thr Thr Thr Ala Cys Ala
        595                 600                 605

Cys Cys Ala Ala Cys Gly Thr Ala Thr Thr Ala Ala Thr Ala Cys Ala
    610                 615                 620

Gly Thr Ala Gly Cys Thr Ala Thr Gly Cys Ala Ala Cys Cys Thr Gly
625                 630                 635                 640

Gly Thr Cys Cys Ala Ala Cys Ala Gly Gly Cys Ala Thr Gly Thr Cys
                645                 650                 655

Ala Gly Gly Thr Cys Cys Thr Cys Ala Ala Gly Gly Thr Thr Thr Ala
            660                 665                 670

Cys Cys Ala Gly Gly Thr Cys Cys Thr Ala Cys Thr Gly Gly Thr Cys
        675                 680                 685

Cys Ala Gly Cys Ala Gly Gly Cys Cys Ala Ala Ala Ala Thr Gly Gly
    690                 695                 700

Thr Thr Cys Thr Cys Cys Ala Gly Gly Cys Thr Cys Ala Cys Cys Ala
705                 710                 715                 720

Gly Gly Ala Gly Cys Ala Ala Thr Gly Gly Gly Thr Cys Cys Ala Cys
                725                 730                 735

Ala Ala Gly Gly Ala Cys Cys Ala Ala Thr Gly Gly Gly Thr Cys Cys
            740                 745                 750

Ala Ala Gly Ala Gly Gly Thr Thr Ala Thr Cys Gly Thr Gly Gly Thr
        755                 760                 765

Thr Thr Ala Cys Gly Thr Gly Gly Thr Cys Cys Ala Cys Cys Thr Gly
    770                 775                 780

Gly Thr Gly Ala Thr Cys Ala Ala Gly Gly Thr Cys Gly Thr Cys Cys
785                 790                 795                 800

Thr Gly Gly Thr Gly Ala Thr Ala Cys Ala Gly Gly Thr Ala Gly Ala
                805                 810                 815

Cys Cys Thr Gly Gly Thr Gly Cys Thr Cys Cys Thr Gly Gly Thr Ala
            820                 825                 830

Thr Thr Cys Cys Ala Gly Gly Thr Cys Ala Ala Ala Thr Thr Gly Gly
        835                 840                 845

Thr Cys Ala Ala Cys Gly Thr Gly Gly Ala Cys Cys Thr Ala Thr Thr
    850                 855                 860

Gly Gly Thr Cys Cys Ala Ala Thr Gly Gly Gly Thr Gly Thr Thr Cys
865                 870                 875                 880

Ala Ala Gly Gly Cys Cys Cys Thr Gly Ala Ala Gly Gly Thr Cys Cys
                885                 890                 895

Ala Cys Gly Thr Gly Gly Thr Ala Ala Thr Cys Cys Thr Gly Gly Ala
            900                 905                 910

Thr Thr Ala Cys Cys Ala Gly Gly Thr Cys Cys Ala Cys Cys Ala Gly
        915                 920                 925

Gly Thr Gly Cys Ala Cys Cys Ala Gly Gly Thr Gly Thr Thr Cys Cys
    930                 935                 940

Ala Gly Gly Thr Ala Thr Thr Gly Gly Thr Ala Thr Thr Cys Ala Ala
945                 950                 955                 960

Gly Gly Ala Cys Cys Ala Gly Cys Ala Gly Gly Thr Cys Cys Thr Cys
                965                 970                 975

Cys Thr Gly Gly Thr Cys Cys Ala Cys Cys Ala Gly Gly Ala Cys Gly
            980                 985                 990

Cys Thr Thr Thr Cys Cys Thr Gly Cys Thr Ala Cys Ala Gly Ala Ala
        995                 1000                1005

Cys Ala Thr Thr Gly Thr Ala Gly Cys Thr Ala Thr Gly Thr Thr
    1010                1015                1020

Ala Cys Thr Gly Gly Thr Gly Ala Ala Thr Gly Thr Gly Thr Ala
    1025                1030                1035

Ala Thr Thr Ala Ala Cys Gly Gly Cys Ala Ala Thr Gly Ala Cys
    1040                1045                1050

Ala Thr Thr Thr Thr Cys Cys Ala Thr Gly Cys Thr Ala Thr Gly
    1055                1060                1065

Cys Gly Thr Gly Ala Ala Ala Cys Thr Gly Gly Cys Gly Ala Thr
    1070                1075                1080

Gly Thr Ala Ala Gly Thr Thr Gly Thr Cys Cys Ala Ala Gly Ala
    1085                1090                1095

Ala Ala Cys Thr Ala Cys Thr Ala Thr Gly Thr Thr Ala Ala Ala
    1100                1105                1110

Gly Gly Thr Gly Thr Thr Gly Ala Thr Thr Ala Cys Ala Thr Cys
    1115                1120                1125

Ala Ala Ala Thr Gly Cys Ala Ala Thr Ala Ala Thr Gly Gly Thr

1130                 1135                 1140

Gly Cys  Thr Gly Ala Ala Thr  Ala Cys Cys Ala Cys  Ala Ala Thr
    1145                 1150                 1155

Ala Cys  Thr Thr Thr Ala Cys  Ala Gly Thr Thr Ala  Cys Gly Thr
    1160                 1165                 1170

Thr Thr  Ala Ala Cys Ala Thr  Gly Thr Thr Gly Thr  Thr Thr Ala
    1175                 1180                 1185

Thr Thr  Ala Gly Gly Thr Thr  Thr Ala Ala Ala Cys  Gly Ala Gly
    1190                 1195                 1200

Ala Ala  Thr Cys Thr Thr Thr  Ala Thr Thr Thr Cys  Cys Ala Ala
    1205                 1210                 1215

Gly Gly  Thr Thr Ala Thr Cys  Cys Thr Thr Ala Thr  Gly Ala Thr
    1220                 1225                 1230

Gly Thr  Thr Cys Cys Ala Gly  Ala Thr Thr Ala Thr  Gly Cys Thr
    1235                 1240                 1245

Thr Ala  Ala
    1250

<210>  11
<211>  18
<212>  DNA
<213>  Artificial Sequence

<220>
<223>  nucleic sequence encoding the elastin peptide VGVAPG

<400>  11
gtaggtgtag ctcctggt                                                    18

<210>  12
<211>  18
<212>  DNA
<213>  Artificial Sequence

<220>
<223>  nucleic sequence encoding the elastin peptide VGVAPG

<400>  12
gttggtgttg ctcctgga                                                    18

<210>  13
<211>  18
<212>  DNA
<213>  Artificial Sequence

<220>
<223>  nucleic sequence encoding the elastin peptide VGVAPG

<400>  13
gtaggtgttg ctccaggt                                                    18


<210>  14
<211>  18
<212>  DNA
<213>  Artificial Sequence

<220>
<223>  nucleic sequence encoding the elastin peptide VGVAPG

<400>  14
gtgggtgtag ctcctggt                                                    18


<210>  15
<211>  21
<212>  DNA
<213>  Artificial Sequence

<220>
<223>  nucleic sequence encoding the elastin peptide derivative VGVAPGE

<400>  15
gtaggtgtag ctcctggtga a                                                21


<210>  16
<211>  21
<212>  DNA
<213>  Artificial Sequence

<220>
<223>  nucleic seuqence encoding the elastin peptide derivative VGVAPGE

<400>  16
gttggtgttg ctcctggaga a                                                21


<210>  17
<211>  21
<212>  DNA
<213>  Artificial Sequence

<220>
<223>  nucleic sequence encoding the elastin peptide derivative VGVAPGE

<400>  17
gtaggtgttg ctccaggtga a                                                21


<210>  18
<211>  21
<212>  DNA
<213>  Artificial Sequence

<220>
<223>  nucleic sequence encoding the elastin peptide derivative VGVAPGE

<400>  18
gtgggtgtag ctcctggtga a                                                21


<210>  19
<211>  852
<212>  DNA
<213>  Artificial Sequence

<220>
<223>  ha-sp-3f-Gtccld-3ha

<400>  19
atggcatatc cttatgatgt accagattat gccaataata acgacttatt tcaagcatca       60

cgtcgtcgtt tcttagctca attaggaggt ttaacagttg ctggtatgtt aggtccatct      120

ttacttactc cacgtagagc tacagcagct caagctgatt acaaagatga tgacgataaa      180

gactataaag atgatgatga caaagattac aaagacgatg atgataaagg atccggtcca      240

tgttgtggac cacctggtcc tccaggtcca ccaggaccac ctggaccaac aggtatgagt      300

ggaccacaag gtttaccagg tcctactggt cctgctggtc aaaacggttc accaggttct      360

ccaggtgcaa tgggtccaca aggtcctatg ggaccacgtg gttatcgtgg tttacgtggt      420

ccaccaggtg atcaaggtcg tcctggtgat acaggtcgtc caggtgctcc aggtattcca      480

ggtcagattg gtcaacgtgg tccaattggt cctatgggtg ttcaaggacc agaaggtcca      540

agaggtaatc caggccttcc aggaccacca ggtgcaccag gtgtacctgg tattggcatt      600

caaggtcctg ctggcccacc tggtccacct ggtcgttttg gtcctcctgg ccctccaggc      660

ccaccaggtc ctcctggtcc atgttgcggc tatatcccag aagctccacg tgatggtcaa      720

gcttatgttc gcaaagatgg tgaatgggtg ttattatcaa cattcttata cccttatgac      780

gtaccagatt acgcatatcc ttatgatgtt ccagattatg cctatccata cgatgtacct      840

gactatgctt aa                                                          852


<210>  20
<211>  771
<212>  DNA
<213>  Artificial Sequence

<220>
<223>  ha-sp-3f-Gtccld

<400>  20
atggcatatc cttatgatgt accagattat gccaataata acgacttatt tcaagcatca       60

cgtcgtcgtt tcttagctca attaggaggt ttaacagttg ctggtatgtt aggtccatct      120

ttacttactc cacgtagagc tacagcagct caagctgatt acaaagatga tgacgataaa      180

gactataaag atgatgatga caaagattac aaagacgatg atgataaagg atccggtcca      240

tgttgtggac cacctggtcc tccaggtcca ccaggaccac ctggaccaac aggtatgagt      300

ggaccacaag gtttaccagg tcctactggt cctgctggtc aaaacggttc accaggttct 360

ccaggtgcaa tgggtccaca aggtcctatg ggaccacgtg gttatcgtgg tttacgtggt 420

ccaccaggtg atcaaggtcg tcctggtgat acaggtcgtc caggtgctcc aggtattcca 480

ggtcagattg gtcaacgtgg tccaattggt cctatgggtg ttcaaggacc agaaggtcca 540

agaggtaatc caggccttcc aggaccacca ggtgcaccag gtgtacctgg tattggcatt 600

caaggtcctg ctggcccacc tggtccacct ggtcgttttg gtcctcctgg ccctccaggc 660

ccaccaggtc ctcctggtcc atgttgcggc tatatcccag aagctccacg tgatggtcaa 720

gcttatgttc gcaaagatgg tgaatgggtg ttattatcaa cattcttata a 771

<210> 21
<211> 594
<212> DNA
<213> Artificial Sequence

<220>
<223> ha-sp-3f-Gtcld

<400> 21
atggcatatc cttatgatgt accagattat gccaataata acgacttatt tcaagcatca 60

cgtcgtcgtt tcttagctca attaggaggt ttaacagttg ctggtatgtt aggtccatct 120

ttacttactc cacgtagagc tacagcagct caagctgatt acaaagatga tgacgataaa 180

gactataaag atgatgatga caaagattac aaagacgatg atgataaagg atccggacca 240

acaggtatga gtggaccaca aggtttacca ggtcctactg gtcctgctgg tcaaaacggt 300

tcaccaggtt ctccaggtgc aatgggtcca caaggtccta tgggaccacg tggttatcgt 360

ggtttacgtg gtccaccagg tgatcaaggt cgtcctggtg atacaggtcg tccaggtgct 420

ccaggtattc caggtcagat tggtcaacgt ggtccaattg gtcctatggg tgttcaagga 480

ccagaaggtc caagaggtaa tccaggcctt ccaggaccac caggtgcacc aggtgtacct 540

ggtattggca ttcaaggtcc tgctggccca cctggtccac ctggtcgttt ttaa 594

<210> 22
<211> 72
<212> DNA
<213> Artificial Sequence

<220>
<223> elp4

<400> 22
gtaggtgtag ctcctggtgt tggtgttgct cctggagtag gtgttgctcc aggtgtgggt 60

gtagctcctg gt 72

<210> 23

<211>  84
<212>  DNA
<213>  Artificial Sequence

<220>
<223>  elpe4

<400>  23
gtaggtgtag ctcctggtga agttggtgtt gctcctggag aagtaggtgt tgctccaggt        60

gaagtgggtg tagctcctgg tgaa                                               84


<210>  24
<211>  8
<212>  PRT
<213>  Artificial Sequence

<220>
<223>  Flag Tag

<400>  24

Asp Tyr Lys Asp Asp Asp Asp Lys
1               5


<210>  25
<211>  24
<212>  PRT
<213>  Artificial Sequence

<220>
<223>  3X Flag Tag

<400>  25

Asp Tyr Lys Asp Asp Asp Asp Lys Asp Tyr Lys Asp Asp Asp Asp Lys
1               5                   10                  15


Asp Tyr Lys Asp Asp Asp Asp Lys
            20


<210>  26
<211>  9
<212>  PRT
<213>  Artificial Sequence

<220>
<223>  HA Tag

<400>  26

Tyr Pro Tyr Asp Val Pro Asp Tyr Ala
1               5


<210>  27
<211>  27
<212>  PRT
<213>  Artificial Sequence

<220>
<223>  3xHA Tag

<400>  27

Tyr Pro Tyr Asp Val Pro Asp Tyr Ala Tyr Pro Tyr Asp Val Pro Asp
1               5                   10                  15

Tyr Ala Tyr Pro Tyr Asp Val Pro Asp Tyr Ala
            20                  25

<210>  28
<211>  6
<212>  PRT
<213>  Artificial Sequence

<220>
<223>  His Tag

<400>  28

His His His His His His
1               5

<210>  29
<211>  424
<212>  DNA
<213>  Artificial Sequence

<220>
<223>  PpsbD-5'UTR

<400>  29
atgaaattaa atggatattt ggtacattta attccacaaa aatgtccaat acttaaaata      60

caaaattaaa agtattagtt gtaaacttga ctaacatttt aaattttaaa tttttcccta     120

attatatatt ttacttgcaa aatttataaa aattttatgc atttttatat cataataata     180

aaacctttat tcatggttta taatataata attgtgatga ctatgcacaa agcagttcta     240

gtcccatata tataactata tataacccgt ttaaagattt atttaaaaat atgtgtgtaa     300

aaaatgctta tttttaattt tattttatat aagttataat attaaataca caatgattaa     360

aattaaataa taataaattt aacgtaacga tgagttgttt ttttattttg gagatacacg     420

cacc                                                                  424

<210>  30
<211>  1025
<212>  DNA
<213>  Artificial Sequence

<220>
<223>  Prrn-5'UTRatpA

<400>  30

tttttaatta agtaggaact cggtatatgc tcttttgggg tcttattagc tagtattagt 60

taactaacaa aagatcaata ttttagtttg ttttatatat tttattactt aagtagtaag 120

gatttgcatt tagcaatctt aaatacttaa gtaataatct ataaataaaa tatattttcg 180

ctttaaaact tataaaaatt atttgctcgt tataagccta aaaaaacgta ggatctctac 240

gagatattac attgtttttt tctttaattg gctttaatat tactttgtat atataaacca 300

aagtacttgt taatagttat taaattatat taactataca gtacaaagaa attttttgct 360

aaaaaaagta tgttaacatt aaaaattttt gtttatacag actagtcaga ctcggggggc 420

aggcaacaaa tttatttatt gtcccgtaag gggaagggga aaacaattat tattttactg 480

cggagcagct tgttattgaa attttattaa aaaaaaaata aaaatttgac aaaaaaaaat 540

aaaaaagtta aattaaaaac actgggaatg ttctacatca taaaaatcaa aagggtttaa 600

aatcccgaca aaatttaaac tttaaagagt ggcgcctacc ttttttttaa tttgcatgat 660

tttaatgctt atgctatctt ttttatttag tccataaaac ctttaaagga ccttttctta 720

tgggatattt atattttcct aacaaagcaa tcggcgtcat aaactttagt tgcttacgac 780

gcctgtggac gtccccccct tccccttacg ggcaagtaaa cttagggatt ttaatgcaat 840

aaataaattt gtcctcttcg ggcaaatgaa ttttagtatt taaatatgac aagggtgaac 900

cattactttt gttaacaagt gatcttacca ctcactattt ttgttgaatt ttaaacttat 960

ttaaaattct cgagaaagat tttaaaaata aactttttta atcttttatt tattttttct 1020

ttttt 1025

<210> 31
<211> 400
<212> DNA
<213> Artificial Sequence

<220>
<223> 3'UTRatpA

<400> 31
tttttaatta agtaggaact cggtatatgc tcttttgggg tcttattagc tagtattagt 60

taactaacaa aagatcaata ttttagtttg ttttatatat tttattactt aagtagtaag 120

gatttgcatt tagcaatctt aaatacttaa gtaataatct ataaataaaa tatattttcg 180

ctttaaaact tataaaaatt atttgctcgt tataagccta aaaaaacgta ggatctctac 240

gagatattac attgtttttt tctttaattg gctttaatat tactttgtat atataaacca 300

aagtacttgt taatagttat taaattatat taactataca gtacaaagaa attttttgct 360

aaaaaaagta tgttaacatt aaaaattttt gtttatacag 400

<210> 32
<211> 435

<212>  DNA
<213>  Artificial Sequence

<220>
<223>  3'UTRrbcL

<400>  32
aagcttgtac tcaagctcgt aacgaaggtc gtgaccttgc tcgtgaaggt ggcgacgtaa      60

ttcgttcagc ttgtaaatgg tctccagaac ttgctgctgc atgtgaagtt tggaaagaaa     120

ttaaattcga atttgatact attgacaaac tttaattttt atttttcatg atgtttatgt     180

gaatagcata aacatcgttt ttatttttta tggtgtttag gttaaatacc taaacatcat     240

tttacatttt taaaattaag ttctaaagtt atcttttgtt taaatttgcc tgtgctttat     300

aaattacgat gtgccagaaa aataaaatct tagcttttta ttatagaatt tatctttatg     360

tattatattt tataagtaat aaaagaaata gtaacatact aaagcggatg taactcaatc     420

ggtagagtgc gatcc                                                      435


<210>  33
<211>  41
<212>  PRT
<213>  Artificial Sequence

<220>
<223>  SP

<400>  33

Asn Asn Asn Asp Leu Phe Gln Ala Ser Arg Arg Arg Phe Leu Ala Gln
1               5                   10                  15


Leu Gly Gly Leu Thr Val Ala Gly Met Leu Gly Pro Ser Leu Leu Thr
            20                  25                  30


Pro Arg Arg Ala Thr Ala Ala Gln Ala
        35                  40


<210>  34
<211>  123
<212>  DNA
<213>  Artificial Sequence

<220>
<223>  nucleic sequence encoding SP

<400>  34
aataataacg acttatttca agcatcacgt cgtcgtttct tagctcaatt aggaggttta      60

acagttgctg gtatgttagg tccatcttta cttactccac gtagagctac agcagctcaa     120

gct                                                                   123


<210>  35

<211>  58
<212>  PRT
<213>  Bos taurus

<400>  35

<210>  36
<211>  174
<212>  DNA
<213>  Bos taurus

<400>  36
cggcctgact tctgcctaga gcctccatat acgggtccct gcaaggccag aattatcaga      60

tacttctaca acgccaaggc tgggctctgc cagacctttg tatatggcgg ctgcagagct     120

aaaagaaaca atttcaagag cgcagaggac tgcatgagga cctgtggtgg tgct           174

<210>  37
<211>  67
<212>  PRT
<213>  Artificial Sequence

<220>
<223>  HA-APRO

<400>  37

Tyr Pro Tyr Asp Val Pro Asp Tyr Ala Arg Pro Asp Phe Cys Leu Glu
1               5                   10                  15

Pro Pro Tyr Thr Gly Pro Cys Lys Ala Arg Ile Ile Arg Tyr Phe Tyr
            20                  25                  30

Asn Ala Lys Ala Gly Leu Cys Gln Thr Phe Val Tyr Gly Gly Cys Arg
        35                  40                  45

Ala Lys Arg Asn Asn Phe Lys Ser Ala Glu Asp Cys Met Arg Thr Cys
    50                  55                  60

Gly Gly Ala
65

<210>  38
<211>  5
<212>  PRT
<213>  Artificial Sequence

<220>
<223>  EK

<400>  38

Asp Asp Asp Asp Lys
1               5


<210>  39
<211>  85
<212>  PRT
<213>  Artificial Sequence

<220>
<223>  3F-APRO

<400>  39

Ala Gln Ala Asp Tyr Lys Asp Asp Asp Asp Lys Asp Tyr Lys Asp Asp
1               5                   10                  15


Asp Asp Lys Asp Tyr Lys Asp Asp Asp Asp Lys Arg Pro Asp Phe Cys
            20                  25                  30


Leu Glu Pro Pro Tyr Thr Gly Pro Cys Lys Ala Arg Ile Ile Arg Tyr
        35                  40                  45


Phe Tyr Asn Ala Lys Ala Gly Leu Cys Gln Thr Phe Val Tyr Gly Gly
    50                  55                  60


Cys Arg Ala Lys Arg Asn Asn Phe Lys Ser Ala Glu Asp Cys Met Arg
65                  70                  75                  80


Thr Cys Gly Gly Ala
                85


<210>  40
<211>  234
<212>  DNA
<213>  Artificial Sequence

<220>
<223>  lgm-tv-elpe4

<400>  40
cagctgaaga ttgtatgcgt acttgtggtg gtgctagatc tggtggaggc ggttcaagtg        60

gtggaggagg tggcggatct tcaagatctg aaaatttata ttttcaaggt gtaggtgtag       120

ctcctggtga agttggtgtt gctcctggag aagtaggtgt tgctccaggt gaagtgggtg 180

tagctcctgg tgaataagtt taaaccaggt gacctgcaga gctagctact gcag 234

<210> 41
<211> 140
<212> PRT
<213> Artificial Sequence

<220>
<223> HA-SP-3F-FX-APRO

<400> 41

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala
    130                 135                 140

<210> 42
<211> 423
<212> DNA
<213> Artificial Sequence

<220>
<223> nucleic sequence coding for HA-SP-3F-FX-APRO

<400> 42
atggcatatc cttatgatgt accagattat gccaataata acgacttatt tcaagcatca 60

cgtcgtcgtt tcttagctca attaggaggt ttaacagttg ctggtatgtt aggtccatct 120

ttacttactc cacgtagagc tacagcagct caagctgatt acaaagatga tgacgataaa 180

gactataaag atgatgatga caaagattac aaagacgatg atgataaagg atccattgaa 240

ggtcgtagac cagacttttg cttagaacca ccatatacag gtccatgtaa agctcgtatc 300

attcgctatt tctacaatgc aaaagcagga ttatgtcaaa catttgtgta tggaggttgt 360

cgtgctaaac gtaacaactt taaatcagct gaagattgta tgcgtacttg tggtggtgct 420

taa 423

<210> 43
<211> 134
<212> PRT
<213> Artificial Sequence

<220>
<223> HA-SP-3F-APRO

<400> 43

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Arg Pro Asp Phe
65                  70                  75                  80

Cys Leu Glu Pro Pro Tyr Thr Gly Pro Cys Lys Ala Arg Ile Ile Arg
                85                  90                  95

Tyr Phe Tyr Asn Ala Lys Ala Gly Leu Cys Gln Thr Phe Val Tyr Gly
            100                 105                 110

Gly Cys Arg Ala Lys Arg Asn Asn Phe Lys Ser Ala Glu Asp Cys Met
        115                 120                 125

Arg Thr Cys Gly Gly Ala
    130

<210> 44
<211> 349
<212> DNA

<213>  Artificial Sequence

<220>
<223>  nucleic sequence encoding HA-SP-3F-APRO

<400>  44
atcacgtcgt cgtttcttag ctcaattagg aggtttaaca gttgctggta tgttaggtcc      60

atctttactt actccacgta gagctacagc agctcaagct gattacaaag atgatgacga     120

taaagactat aaagatgatg atgacaaaga ttacaaagac gatgatgata aaagaccaga      180

cttttgctta gaaccaccat atacaggtcc atgtaaagct cgtatcattc gctatttcta     240

caatgcaaaa gcaggattat gtcaaacatt tgtgtatgga ggttgtcgtg ctaaacgtaa     300

caactttaaa tcagctgaag attgtatgcg tacttgtggt ggtgcttaa                 349


<210>  45
<211>  108
<212>  PRT
<213>  Artificial Sequence

<220>
<223>  HA-SP-APRO

<400>  45

Tyr Pro Tyr Asp Val Pro Asp Tyr Ala Asn Asn Asn Asp Leu Phe Gln
1               5                   10                  15


Ala Ser Arg Arg Arg Phe Leu Ala Gln Leu Gly Gly Leu Thr Val Ala
            20                  25                  30


Gly Met Leu Gly Pro Ser Leu Leu Thr Pro Arg Arg Ala Thr Ala Ala
        35                  40                  45


Gln Ala Arg Pro Asp Phe Cys Leu Glu Pro Pro Tyr Thr Gly Pro Cys
    50                  55                  60


Lys Ala Arg Ile Ile Arg Tyr Phe Tyr Asn Ala Lys Ala Gly Leu Cys
65                  70                  75                  80


Gln Thr Phe Val Tyr Gly Gly Cys Arg Ala Lys Arg Asn Asn Phe Lys
                85                  90                  95


Ser Ala Glu Asp Cys Met Arg Thr Cys Gly Gly Ala
            100                 105


<210>  46
<211>  324
<212>  DNA
<213>  Artificial Sequence

<220>

<223>  nucleic sequence encoding for HA-SP-APRO

<400>  46
tatccttatg atgtaccaga ttatgccaat aataacgact tatttcaagc atcacgtcgt      60

cgtttcttag ctcaattagg aggtttaaca gttgctggta tgttaggtcc atctttactt     120

actccacgta gagctacagc agctcaagct agaccagact tttgcttaga accaccatat     180

acaggtccat gtaaagctcg tatcattcgc tatttctaca atgcaaaagc aggattatgt     240

caaacatttg tgtatggagg ttgtcgtgct aaacgtaaca actttaaatc agctgaagat     300

tgtatgcgta cttgtggtgg tgct                                            324


<210>  47
<211>  91
<212>  PRT
<213>  Artificial Sequence

<220>
<223>  3F-FX-APRO

<400>  47

Ala Gln Ala Asp Tyr Lys Asp Asp Asp Asp Lys Asp Tyr Lys Asp Asp
1               5                   10                  15


Asp Asp Lys Asp Tyr Lys Asp Asp Asp Asp Lys Gly Ser Ile Glu Gly
            20                  25                  30


Arg Arg Pro Asp Phe Cys Leu Glu Pro Pro Tyr Thr Gly Pro Cys Lys
        35                  40                  45


Ala Arg Ile Ile Arg Tyr Phe Tyr Asn Ala Lys Ala Gly Leu Cys Gln
    50                  55                  60


Thr Phe Val Tyr Gly Gly Cys Arg Ala Lys Arg Asn Asn Phe Lys Ser
65                  70                  75                  80


Ala Glu Asp Cys Met Arg Thr Cys Gly Gly Ala
                85                  90


<210>  48
<211>  231
<212>  DNA
<213>  Artificial Sequence

<220>
<223>  lgm-ek-elpe4

<400>  48
cagctgaaga ttgtatgcgt acttgtggtg gtgctagatc tggtggaggc ggttcaagtg      60

gtggaggagg tggcggatct tcaagatctg acgatgacga caagggtgta ggtgtagctc     120

ctggtgaagt tggtgttgct cctggagaag taggtgttgc tccaggtgaa gtgggtgtag 180

ctcctggtga ataagtttaa accaggtgac ctgcagagct agctactgca g 231

<210> 49
<211> 14
<212> PRT
<213> Artificial Sequence

<220>
<223> LG

<400> 49

Arg Ser Gly Gly Gly Gly Ser Gly Gly Gly Gly Ser Gly Ser
1               5                   10

<210> 50
<211> 18
<212> PRT
<213> Artificial Sequence

<220>
<223> LGM

<400> 50

Arg Ser Gly Gly Gly Gly Ser Ser Gly Gly Gly Gly Gly Gly Ser Ser
1               5                   10                  15

Arg Ser

<210> 51
<211> 365
<212> PRT
<213> Artificial Sequence

<220>
<223> CCMP2712

<400> 51

Met Ser Arg Arg Arg Gln Gly Ala Ala Ala Ala Ala Ala Gly Ala Ala
1               5                   10                  15

Gly Leu Ile Leu Leu Val Leu Leu Ser Leu Val Ser Leu Arg Ala His
            20                  25                  30

Glu Arg Lys Pro Thr Ser Leu Val Glu Gly Ser Arg Thr Thr Met Met
        35                  40                  45

Pro Thr Trp Lys Val Thr Gly Thr Leu Asn Pro Val Glu Glu Thr Glu
    50                  55                  60

Lys Glu Val Glu Glu Glu Pro Ser Ser Pro Pro Pro Pro Ala Val Gln
65                  70                  75                  80

Ala Thr Pro Asp Val Pro Gln Glu Gln Glu Ala Glu Pro Ser Gln Ser
                85                  90                  95

Glu Trp Val Pro Pro Pro Lys Pro Trp Glu Pro Leu Ala Glu Lys Asn
            100                 105                 110

Ile Gly Arg Leu Glu Arg Thr Asn Arg Glu Val Val Arg Gly Asp Met
        115                 120                 125

Glu Asn Ala Asn Met Ile Lys Lys Leu Arg Ala Ala Met Arg Leu Leu
    130                 135                 140

Lys Lys Gln Phe Ala Ala Lys Ile Val Asn Leu Glu Gln Ala Tyr Asp
145                 150                 155                 160

Arg Arg Leu Ala Arg Glu Ser Arg Ile Leu His Gln Arg Ile Asn Thr
                165                 170                 175

Val Ala Met Gln Pro Gly Pro Thr Gly Met Ser Gly Pro Gln Gly Leu
            180                 185                 190

Pro Gly Pro Thr Gly Pro Ala Gly Gln Asn Gly Ser Pro Gly Ser Pro
        195                 200                 205

Gly Ala Met Gly Pro Gln Gly Pro Met Gly Pro Arg Gly Tyr Arg Gly
    210                 215                 220

Leu Arg Gly Pro Pro Gly Asp Gln Gly Arg Pro Gly Asp Thr Gly Arg
225                 230                 235                 240

Pro Gly Ala Pro Gly Ile Pro Gly Gln Ile Gly Gln Arg Gly Pro Ile
                245                 250                 255

Gly Pro Met Gly Val Gln Gly Pro Glu Gly Pro Arg Gly Asn Pro Gly
            260                 265                 270

Leu Pro Gly Pro Pro Gly Ala Pro Gly Val Pro Gly Ile Gly Ile Gln
        275                 280                 285

Gly Pro Ala Gly Pro Pro Gly Pro Pro Gly Arg Phe Pro Ala Thr Glu
    290                 295                 300

His Cys Ser Tyr Val Thr Gly Glu Cys Val Ile Asn Gly Asn Asp Ile
305                 310                 315                 320

Phe His Ala Met Arg Glu Thr Gly Asp Val Ser Cys Pro Arg Asn Tyr
                325                 330                 335

Tyr Val Lys Gly Val Asp Tyr Ile Lys Cys Asn Asn Gly Ala Glu Tyr
            340                 345                 350

His Asn Thr Leu Gln Leu Arg Leu Thr Cys Cys Leu Leu
        355                 360                 365

<210>  52
<211>  7
<212>  PRT
<213>  Artificial Sequence

<220>
<223>  TEV protease

<400>  52

Glu Asn Leu Tyr Phe Gln Gly
1               5

<210>  53
<211>  29
<212>  PRT
<213>  Artificial Sequence

<220>
<223>  polypeptide of example 3

<400>  53

Gly Val Gly Val Ala Pro Gly Glu Val Gly Val Ala Pro Gly Glu Val
1               5                   10                  15

Gly Val Ala Pro Gly Glu Val Gly Val Ala Pro Gly Glu
            20                  25

<210>  54
<211>  34
<212>  DNA
<213>  Artificial Sequence

<220>
<223>  O3'SUTRpsbD

<400>  54
cgatgagttg ttttttattt ttggagatac acgc                                34

<210>  55
<211>  416
<212>  PRT
<213>  Artificial Sequence

<220>

<223>  3F-TV-GtCLP-TV-HA

<400>  55

Met Ala Asp Tyr Lys Asp Asp Asp Asp Lys Asp Tyr Lys Asp Asp Asp
1               5                   10                  15

Asp Lys Asp Tyr Lys Asp Asp Asp Asp Lys Glu Asn Leu Tyr Phe Gln
            20                  25                  30

Gly Ser Arg Arg Arg Gln Gly Ala Ala Ala Ala Ala Ala Gly Ala Ala
        35                  40                  45

Gly Leu Ile Leu Leu Val Leu Leu Ser Leu Val Ser Leu Arg Ala His
    50                  55                  60

Glu Arg Lys Pro Thr Ser Leu Val Glu Gly Ser Arg Thr Thr Met Met
65                  70                  75                  80

Pro Thr Trp Lys Val Thr Gly Thr Leu Asn Pro Val Glu Glu Thr Glu
                85                  90                  95

Lys Glu Val Glu Glu Glu Pro Ser Ser Pro Pro Pro Pro Ala Val Gln
            100                 105                 110

Ala Thr Pro Asp Val Pro Gln Glu Gln Glu Ala Glu Pro Ser Gln Ser
        115                 120                 125

Glu Trp Val Pro Pro Pro Lys Pro Trp Glu Pro Leu Ala Glu Lys Asn
    130                 135                 140

Ile Gly Arg Leu Glu Arg Thr Asn Arg Glu Val Val Arg Gly Asp Met
145                 150                 155                 160

Glu Asn Ala Asn Met Ile Lys Lys Leu Arg Ala Ala Met Arg Leu Leu
                165                 170                 175

Lys Lys Gln Phe Ala Ala Lys Ile Val Asn Leu Glu Gln Ala Tyr Asp
            180                 185                 190

Arg Arg Leu Ala Arg Glu Ser Arg Ile Leu His Gln Arg Ile Asn Thr
        195                 200                 205

Val Ala Met Gln Pro Gly Pro Thr Gly Met Ser Gly Pro Gln Gly Leu
    210                 215                 220

Pro Gly Pro Thr Gly Pro Ala Gly Gln Asn Gly Ser Pro Gly Ser Pro
225                 230                 235                 240

Gly Ala Met Gly Pro Gln Gly Pro Met Gly Pro Arg Gly Tyr Arg Gly
                245                 250                 255

Leu Arg Gly Pro Pro Gly Asp Gln Gly Arg Pro Gly Asp Thr Gly Arg
            260                 265                 270

Pro Gly Ala Pro Gly Ile Pro Gly Gln Ile Gly Gln Arg Gly Pro Ile
        275                 280                 285

Gly Pro Met Gly Val Gln Gly Pro Glu Gly Pro Arg Gly Asn Pro Gly
    290                 295                 300

Leu Pro Gly Pro Pro Gly Ala Pro Gly Val Pro Gly Ile Gly Ile Gln
305                 310                 315                 320

Gly Pro Ala Gly Pro Pro Gly Pro Pro Gly Arg Phe Pro Ala Thr Glu
                325                 330                 335

His Cys Ser Tyr Val Thr Gly Glu Cys Val Ile Asn Gly Asn Asp Ile
            340                 345                 350

Phe His Ala Met Arg Glu Thr Gly Asp Val Ser Cys Pro Arg Asn Tyr
        355                 360                 365

Tyr Val Lys Gly Val Asp Tyr Ile Lys Cys Asn Asn Gly Ala Glu Tyr
    370                 375                 380

His Asn Thr Leu Gln Leu Arg Leu Thr Cys Cys Leu Leu Gly Leu Asn
385                 390                 395                 400

Glu Asn Leu Tyr Phe Gln Gly Tyr Pro Tyr Asp Val Pro Asp Tyr Ala
                405                 410                 415

<210>  56
<211>  62
<212>  DNA
<213>  Artificial Sequence

<220>
<223>  O5'SCL70

<400>  56
ctgcagtagc tagctctgca ggtcacctgt taagcataat ctggaacatc ataaggataa      60

cc                                                                      62

<210>  57
<211>  62
<212>  DNA
<213>  Artificial Sequence

<220>
<223>  O3'SCL70

<400>  57
cgatgagttg tttttttatt ttggagatac acgcaccatg gcagattata aagacgatga      60

tg                                                                    62


<210>  58
<211>  1317
<212>  DNA
<213>  Artificial Sequence

<220>
<223>  FPCR-SCL70

<400>  58
ctgcagtagc tagctctgca ggtcacctgt taagcataat ctggaacatc ataaggataa      60

ccttggaaat aaagattctc gtttaaacct aataaacaac atgttaaacg taactgtaaa     120

gtattgtggt attcagcacc attattgcat ttgatgtaat caacaccttt aacatagtag     180

tttcttggac aacttacatc gccagtttca cgcatagcat ggaaaatgtc attgccgtta     240

attacacatt caccagtaac atagctacaa tgttctgtag caggaaagcg tcctggtgga     300

ccaggaggac ctgctggtcc ttgaatacca atacctggaa cacctggtgc acctggtgga     360

cctggtaatc caggattacc acgtggacct tcagggcctt gaacacccat tggaccaata     420

ggtccacgtt gaccaatttg acctggaata ccaggagcac caggtctacc tgtatcacca     480

ggacgacctt gatcaccagg tggaccacgt aaaccacgat aacctcttgg acccattggt     540

ccttgtggac ccattgctcc tggtgagcct ggagaaccat tttggcctgc tggaccagta     600

ggacctggta aaccttgagg acctgacatg cctgttggac caggttgcat agctactgta     660

ttaatacgtt ggtgtaaaat acgactttca cgagctaaac gacgatcata agcttgttct     720

aaattcacaa ttttagctgc aaattgtttc tttaataaac gcatagcagc acgtaatttt     780

tttatcatgt ttgcattttc catatcacca cgaactactt cacgattagt acgttctaaa     840

cgaccaatat ttttttcagc taatggttcc caaggtttag gtggtggaac ccattctgac     900

tgacttggtt cggcttcttg ctcttgaggt acatctggtg tagcttgtac tgctggtggt     960

ggaggtgaag atggttcttc ttctacttct ttctctgttt cttcaactgg atttaatgta    1020

ccagttactt tccatgttgg catcattgta gtgcgtgaac cttcaactaa agatgttggt    1080

ttgcgttcat gagcacgtaa agatactaat gaaagtaaca caagtaagat taatcctgct    1140

gcaccagcgg ctgcggcagc agcaccttga cgacgacgac taccttgaaa ataaaggttt    1200

tctttatcgt catcatcttt atagtctttg tcgtcatcgt ctttgtaatc tttatcatca    1260

tcgtctttat aatctgccat ggtgcgtgta tctccaaaat aaaaaaacaa ctcatcg       1317

<210>  59
<211>  119
<212>  PRT
<213>  Artificial Sequence

<220>
<223>  collagen-like domain from G. theta CCMP2712

<400>  59

Gly Pro Thr Gly Met Ser Gly Pro Gln Gly Leu Pro Gly Pro Thr Gly
1               5                   10                  15

Pro Ala Gly Gln Asn Gly Ser Pro Gly Ser Pro Gly Ala Met Gly Pro
            20                  25                  30

Gln Gly Pro Met Gly Pro Arg Gly Tyr Arg Gly Leu Arg Gly Pro Pro
        35                  40                  45

Gly Asp Gln Gly Arg Pro Gly Asp Thr Gly Arg Pro Gly Ala Pro Gly
    50                  55                  60

Ile Pro Gly Gln Ile Gly Gln Arg Gly Pro Ile Gly Pro Met Gly Val
65                  70                  75                  80

Gln Gly Pro Glu Gly Pro Arg Gly Asn Pro Gly Leu Pro Gly Pro Pro
                85                  90                  95

Gly Ala Pro Gly Val Pro Gly Ile Gly Ile Gln Gly Pro Ala Gly Pro
            100                 105                 110

Pro Gly Pro Pro Gly Arg Phe
        115


<210>  60
<211>  16
<212>  PRT
<213>  Artificial Sequence

<220>
<223>  Cys Knot and CR4 Repeat sequences

<400>  60

Gly Pro Cys Cys Gly Pro Pro Gly Pro Pro Gly Pro Pro Gly Pro Pro
1               5                   10                  15


<210>  61
<211>  27
<212>  PRT
<213>  Artificial Sequence

<220>

<223>  Foldon fibritin of the T4 phage

<400>  61

Gly Tyr Ile Pro Glu Ala Pro Arg Asp Gly Gln Ala Tyr Val Arg Lys
1               5                   10                  15

Asp Gly Glu Trp Val Leu Leu Ser Thr Phe Leu
            20                  25

<210>  62
<211>  180
<212>  PRT
<213>  Artificial Sequence

<220>
<223>  GtCCLD

<400>  62

Gly Ser Gly Pro Cys Cys Gly Pro Pro Gly Pro Pro Gly Pro Pro Gly
1               5                   10                  15

Pro Pro Gly Pro Thr Gly Met Ser Gly Pro Gln Gly Leu Pro Gly Pro
            20                  25                  30

Thr Gly Pro Ala Gly Gln Asn Gly Ser Pro Gly Ser Pro Gly Ala Met
        35                  40                  45

Gly Pro Gln Gly Pro Met Gly Pro Arg Gly Tyr Arg Gly Leu Arg Gly
    50                  55                  60

Pro Pro Gly Asp Gln Gly Arg Pro Gly Asp Thr Gly Arg Pro Gly Ala
65                  70                  75                  80

Pro Gly Ile Pro Gly Gln Ile Gly Gln Arg Gly Pro Ile Gly Pro Met
                85                  90                  95

Gly Val Gln Gly Pro Glu Gly Pro Arg Gly Asn Pro Gly Leu Pro Gly
            100                 105                 110

Pro Pro Gly Ala Pro Gly Val Pro Gly Ile Gly Ile Gln Gly Pro Ala
        115                 120                 125

Gly Pro Pro Gly Pro Pro Gly Arg Phe Gly Pro Pro Gly Pro Pro Gly
    130                 135                 140

Pro Pro Gly Pro Pro Gly Pro Cys Cys Gly Tyr Ile Pro Glu Ala Pro
145                 150                 155                 160

Arg Asp Gly Gln Ala Tyr Val Arg Lys Asp Gly Glu Trp Val Leu Leu

165                 170                 175

Ser Thr Phe Leu
            180

<210>  63
<211>  27
<212>  PRT
<213>  Artificial Sequence

<220>
<223>  3HA

<400>  63

Tyr Pro Tyr Asp Val Pro Asp Tyr Ala Tyr Pro Tyr Asp Val Pro Asp
1               5                   10                  15

Tyr Ala Tyr Pro Tyr Asp Val Pro Asp Tyr Ala
            20                  25

<210>  64
<211>  207
<212>  PRT
<213>  Artificial Sequence

<220>
<223>  GtCCLD-3HA

<400>  64

Gly Ser Gly Pro Cys Cys Gly Pro Pro Gly Pro Pro Gly Pro Pro Gly
1               5                   10                  15

Pro Pro Gly Pro Thr Gly Met Ser Gly Pro Gln Gly Leu Pro Gly Pro
            20                  25                  30

Thr Gly Pro Ala Gly Gln Asn Gly Ser Pro Gly Ser Pro Gly Ala Met
        35                  40                  45

Gly Pro Gln Gly Pro Met Gly Pro Arg Gly Tyr Arg Gly Leu Arg Gly
    50                  55                  60

Pro Pro Gly Asp Gln Gly Arg Pro Gly Asp Thr Gly Arg Pro Gly Ala
65                  70                  75                  80

Pro Gly Ile Pro Gly Gln Ile Gly Gln Arg Gly Pro Ile Gly Pro Met
                85                  90                  95

Gly Val Gln Gly Pro Glu Gly Pro Arg Gly Asn Pro Gly Leu Pro Gly
            100                 105                 110

Pro Pro Gly Ala Pro Gly Val Pro Gly Ile Gly Ile Gln Gly Pro Ala
        115                 120                 125

Gly Pro Pro Gly Pro Pro Gly Arg Phe Gly Pro Pro Gly Pro Pro Gly
    130                 135                 140

Pro Pro Gly Pro Pro Gly Pro Cys Cys Gly Tyr Ile Pro Glu Ala Pro
145                 150                 155                 160

Arg Asp Gly Gln Ala Tyr Val Arg Lys Asp Gly Glu Trp Val Leu Leu
                165                 170                 175

Ser Thr Phe Leu Tyr Pro Tyr Asp Val Pro Asp Tyr Ala Tyr Pro Tyr
            180                 185                 190

Asp Val Pro Asp Tyr Ala Tyr Pro Tyr Asp Val Pro Asp Tyr Ala
        195                 200                 205

<210>  65
<211>  617
<212>  DNA
<213>  Artificial Sequence

<220>
<223>  PCR fragment Gtccld-2

<400>  65
gatgatgatg acaaagatta caaagacgat gatgataaag gatccggtcc atgttgtgga      60

ccacctggtc ctccaggtcc accaggacca cctggaccaa caggtatgag tggaccacaa     120

ggtttaccag gtcctactgg tcctgctggt caaaacggtt caccaggttc tccaggtgca     180

atgggtccac aaggtcctat gggaccacgt ggttatcgtg gtttacgtgg tccaccaggt     240

gatcaaggtc gtcctggtga tacaggtcgt ccaggtgctc caggtattcc aggtcagatt     300

ggtcaacgtg gtccaattgg tcctatgggt gttcaaggac cagaaggtcc aagaggtaat     360

ccaggccttc caggaccacc aggtgcacca ggtgtacctg gtattggcat tcaaggtcct     420

gctggcccac ctggtccacc tggtcgtttt ggtcctcctg gccctccagg cccaccaggt     480

cctcctggtc catgttgcgg ctatatccca gaagctccac gtgatggtca agcttatgtt     540

cgcaaagatg gtgaatgggt gttattatca acattcttat aagtttaaac gtcgacctgc     600

agagctagct actgcag                                                    617

<210>  66
<211>  440
<212>  DNA
<213>  Artificial Sequence

<220>
<223>  PCR fragment Gtcld

<400>  66
gatgatgatg acaaagatta caaagacgat gatgataaag gatccggacc aacaggtatg      60

agtggaccac aaggtttacc aggtcctact ggtcctgctg gtcaaaacgg ttcaccaggt     120

tctccaggtg caatgggtcc acaaggtcct atgggaccac gtggttatcg tggtttacgt     180

ggtccaccag gtgatcaagg tcgtcctggt gatacaggtc gtccaggtgc tccaggtatt     240

ccaggtcaga ttggtcaacg tggtccaatt ggtcctatgg gtgttcaagg accagaaggt     300

ccaagaggta atccaggcct tccaggacca ccaggtgcac caggtgtacc tggtattggc     360

attcaaggtc ctgctggccc acctggtcca cctggtcgtt tttaagttta aacgtcgacc     420

tgcagagcta gctactgcag                                                 440


<210>  67
<211>  35
<212>  DNA
<213>  Artificial Sequence

<220>
<223>  O5'ASTatpA2

<400>  67
cctacttaat taaaaactgc agtagctagc tctgc                                 35


<210>  68
<211>  34
<212>  DNA
<213>  Artificial Sequence

<220>
<223>  O3'SUTRpsbD

<400>  68
cgatgagttg tttttttatt ttggagatac acgc                                  34


<210>  69
<211>  62
<212>  DNA
<213>  Artificial Sequence

<220>
<223>  O5'SCL70

<400>  69
ctgcagtagc tagctctgca ggtcacctgt taagcataat ctggaacatc ataaggataa      60

cc                                                                     62


<210>  70
<211>  33
<212>  DNA
<213>  Artificial Sequence

<220>

<223>  O5'PpsbDCla2

<400>  70
catcgatgat gaaattaaat ggatatttgg tac                                    33


<210>  71
<211>  4
<212>  PRT
<213>  Artificial Sequence

<220>
<223>  FX

<400>  71

Ile Glu Gly Arg
1


<210>  72
<211>  116
<212>  DNA
<213>  Artificial Sequence

<220>
<223>  O5'Gibs-ELP4

<400>  72
ctgcagtagc tagctctgca ggtcgacgtt taaacttaag cgtaatctgg aacatcatat      60

ggataaccag gagctacacc cacacctgga gcaacaccta ctccaggagc aacacc          116


<210>  73
<211>  110
<212>  DNA
<213>  Artificial Sequence

<220>
<223>  O3'Gibs-ELP4

<400>  73
gctgaagatt gtatgcgtac ttgtggtggt gctgattaca aagacgatga tgacaaagta      60

ggtgtagctc ctggtgttgg tgttgctcct ggagtaggtg ttgctccagg                 110


<210>  74
<211>  62
<212>  DNA
<213>  Artificial Sequence

<220>
<223>  O5'Gibs01BE

<400>  74
ctgcagtagc tagctctgca ggtcgacgtt taaacttaac caggagctac acccacacct      60

gg                                                                      62


<210>  75

<211>  70
<212>  DNA
<213>  Artificial Sequence

<220>
<223>  O3'Gibs01BE

<400>  75
gatgacaaag attacaaaga cgatgatgat aaaggatcca ttgaaggtcg tagaccagac        60

ttttgcttag                                                               70


<210>  76
<211>  359
<212>  DNA
<213>  Artificial Sequence

<220>
<223>  FPCR-AP-FELP4

<400>  76
gatgacaaag attacaaaga cgatgatgat aaaggatcca ttgaaggtcg tagaccagac        60

ttttgcttag aaccaccata tacaggtcca tgtaaagctc gtatcattcg ctatttctac       120

aatgcaaaag caggattatg tcaaacattt gtgtatggag gttgtcgtgc taaacgtaac       180

aactttaaat cagctgaaga ttgtatgcgt acttgtggtg gtgctgatta caaagacgat       240

gatgacaaag taggtgtagc tcctggtgtt ggtgttgctc ctggagtagg tgttgctcca       300

ggtgtgggtg tagctcctgg ttaagtttaa acgtcgacct gcagagctag ctactgcag        359


<210>  77
<211>  207
<212>  DNA
<213>  Artificial Sequence

<220>
<223>  FPCR-FELP4-HA

<400>  77
agatgatgat gacaaagatt acaaagacga tgatgataaa ggatccgatt acaaagacga        60

tgatgacaaa gtaggtgtag ctcctggtgt tggtgttgct cctggagtag gtgttgctcc       120

aggtgtgggt gtagctcctg gttatccata tgatgttcca gattacgctt aagtttaaac       180

gtcgacctgc agagctagct actgcag                                           207


<210>  78
<211>  62
<212>  DNA
<213>  Artificial Sequence

<220>
<223>  O5'Gibs02BE

<400>  78
ctgcagtagc tagctctgca ggtcgacgtt taaacttaag cgtaatctgg aacatcatat        60

gg                                                                    62

<210>  79
<211>  75
<212>  DNA
<213>  Artificial Sequence

<220>
<223>   O3'Gibs02BE

<400>  79
agatgatgat gacaaagatt acaaagacga tgatgataaa ggatccgatt acaaagacga      60

tgatgacaaa gtagg                                                       75

<210>  80
<211>  28
<212>  PRT
<213>  Artificial Sequence

<220>
<223>  ELPE4

<400>  80

Val Gly Val Ala Pro Gly Glu Val Gly Val Ala Pro Gly Glu Val Gly
1               5                   10                  15

Val Ala Pro Gly Glu Val Gly Val Ala Pro Gly Glu
            20                  25

<210>  81
<211>  24
<212>  PRT
<213>  Artificial Sequence

<220>
<223>  ELP4

<400>  81

Val Gly Val Ala Pro Gly Val Gly Val Ala Pro Gly Val Gly Val Ala
1               5                   10                  15

Pro Gly Val Gly Val Ala Pro Gly
            20

<210>  82
<211>  1282
<212>  DNA
<213>  Artificial Sequence

<220>
<223>  expression cassette of example 3

<400>  82
   atgaaattaa atggatattt ggtacattta attccacaaa aatgtccaat acttaaaata 60
   caaaattaaa agtattagtt gtaaacttga ctaacatttt aaattttaaa ttttttccta 120
   attatatatt ttacttgcaa aatttataaa aattttatgc atttttatat cataataata 180
   aaacctttat tcatggttta taatataata attgtgatga ctatgcacaa agcagttcta 240
   gtcccatata tataactata tataacccgt ttaaagattt atttaaaaat atgtgtgtaa 300
   aaaatgctta tttttaattt tattttatat aagttataat attaaataca caatgattaa 360
   aattaaataa taataaattt aacgtaacga tgagttgttt ttttattttg gagatacacg 420
   caccatggca tatccttatg atgtaccaga ttatgccaat aataacgact tatttcaagc 480
   atcacgtcgt cgtttcttag ctcaattagg aggtttaaca gttgctggta tgttaggtcc 540
   atctttactt actccacgta gagctacagc agctcaagct gattacaaag atgatgacga 600
   taaagactat aaagatgatg atgacaaaga ttacaaagac gatgatgata aaggatccat 660
   tgaaggtcgt agaccagact tttgcttaga accaccatat acaggtccat gtaaagctcg 720
   tatcattcgc tatttctaca atgcaaaagc aggattatgt caaacatttg tgtatggagg 780
   ttgtcgtgct aaacgtaaca actttaaatc agctgaagat tgtatgcgta cttgtggtgg 840
   tgcttaagtt taaacgtcga cctgcagagc tagctactgc agtttttaat taagtaggaa 900
   ctcggtatat gctcttttgg ggtcttatta gctagtatta gttaactaac aaaagatcaa 960
   tattttagtt tgttttatat attttattac ttaagtagta aggatttgca tttagcaatc 1020
   ttaaatactt aagtaataat ctataaataa aatatatttt cgctttaaaa cttataaaaa 1080
   ttatttgctc gttataagcc taaaaaaacg taggatctct acgagatatt acattgtttt 1140
   tttctttaat tggctttaat attactttgt atatataaac caaagtactt gttaatagtt 1200
   attaaattat attaactata cagtacaaag aaattttttg ctaaaaaaag tatgttaaca 1260
   ttaaaaattt ttgtttatac ag 1282

<210> 83
<211> 283
<212> PRT
<213> Artificial Sequence

<220>
<223> HA-SP-3F-GtCCLD-3HA

<400> 83

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Gly Pro
65                  70                  75                  80

Cys Cys Gly Pro Pro Gly Pro Pro Gly Pro Pro Gly Pro Pro Gly Pro
                85                  90                  95

Thr Gly Met Ser Gly Pro Gln Gly Leu Pro Gly Pro Thr Gly Pro Ala
            100                 105                 110

Gly Gln Asn Gly Ser Pro Gly Ser Pro Gly Ala Met Gly Pro Gln Gly
        115                 120                 125

Pro Met Gly Pro Arg Gly Tyr Arg Gly Leu Arg Gly Pro Pro Gly Asp
    130                 135                 140

Gln Gly Arg Pro Gly Asp Thr Gly Arg Pro Gly Ala Pro Gly Ile Pro
145                 150                 155                 160

Gly Gln Ile Gly Gln Arg Gly Pro Ile Gly Pro Met Gly Val Gln Gly
                165                 170                 175

Pro Glu Gly Pro Arg Gly Asn Pro Gly Leu Pro Gly Pro Pro Gly Ala
            180                 185                 190

Pro Gly Val Pro Gly Ile Gly Ile Gln Gly Pro Ala Gly Pro Pro Gly
        195                 200                 205

Pro Pro Gly Arg Phe Gly Pro Pro Gly Pro Pro Gly Pro Pro Gly Pro
    210                 215                 220

Pro Gly Pro Cys Cys Gly Tyr Ile Pro Glu Ala Pro Arg Asp Gly Gln
225                 230                 235                 240

Ala Tyr Val Arg Lys Asp Gly Glu Trp Val Leu Leu Ser Thr Phe Leu
                245                 250                 255

Tyr Pro Tyr Asp Val Pro Asp Tyr Ala Tyr Pro Tyr Asp Val Pro Asp
            260                 265                 270

Tyr Ala Tyr Pro Tyr Asp Val Pro Asp Tyr Ala
        275                 280

<210>  84
<211>  652
<212>  DNA
<213>  Artificial Sequence

<220>
<223>  synthetic codon optimized gene Gtccld-3ha

<400>  84
gtccatggct ggatccggtc catgttgtgg accacctggt cctccaggtc caccaggacc      60

acctggacca acaggtatga gtggaccaca aggtttacca ggtcctactg gtcctgctgg     120

tcaaaacggt tcaccaggtt ctccaggtgc aatgggtcca caaggtccta tgggaccacg     180

tggttatcgt ggtttacgtg gtccaccagg tgatcaaggt cgtcctggtg atacaggtcg     240

tccaggtgct ccaggtattc caggtcagat tggtcaacgt ggtccaattg gtcctatggg     300

tgttcaagga ccagaaggtc caagaggtaa tccaggcctt ccaggaccac caggtgcacc     360

aggtgtacct ggtattggca ttcaaggtcc tgctggccca cctggtccac ctggtcgttt     420

tggtcctcct ggccctccag gcccaccagg tcctcctggt ccatgttgcg gctatatccc     480

agaagctcca cgtgatggtc aagcttatgt tcgcaaagat ggtgaatggg tgttattatc     540

aacattctta tacccttatg acgtaccaga ttacgcatat ccttatgatg ttccagatta     600

tgcctatcca tacgatgtac ctgactatgc ttaagtttaa acgtcgacct gc             652


<210>  85
<211>  256
<212>  PRT
<213>  Artificial Sequence

<220>
<223>  HA-SP-3F-GtCCLD

<400>  85

Met Ala Tyr Pro Tyr Asp Val Pro Asp Tyr Ala Asn Asn Asn Asp Leu
1               5                   10                  15


Phe Gln Ala Ser Arg Arg Arg Phe Leu Ala Gln Leu Gly Gly Leu Thr
            20                  25                  30


Val Ala Gly Met Leu Gly Pro Ser Leu Leu Thr Pro Arg Arg Ala Thr
        35                  40                  45


Ala Ala Gln Ala Asp Tyr Lys Asp Asp Asp Asp Lys Asp Tyr Lys Asp
    50                  55                  60


Asp Asp Asp Lys Asp Tyr Lys Asp Asp Asp Asp Lys Gly Ser Gly Pro
65                  70                  75                  80

Cys Cys Gly Pro Pro Gly Pro Pro Gly Pro Pro Gly Pro Pro Gly Pro
                85                  90                  95

Thr Gly Met Ser Gly Pro Gln Gly Leu Pro Gly Pro Thr Gly Pro Ala
            100                 105                 110

Gly Gln Asn Gly Ser Pro Gly Ser Pro Gly Ala Met Gly Pro Gln Gly
        115                 120                 125

Pro Met Gly Pro Arg Gly Tyr Arg Gly Leu Arg Gly Pro Pro Gly Asp
    130                 135                 140

Gln Gly Arg Pro Gly Asp Thr Gly Arg Pro Gly Ala Pro Gly Ile Pro
145                 150                 155                 160

Gly Gln Ile Gly Gln Arg Gly Pro Ile Gly Pro Met Gly Val Gln Gly
                165                 170                 175

Pro Glu Gly Pro Arg Gly Asn Pro Gly Leu Pro Gly Pro Pro Gly Ala
            180                 185                 190

Pro Gly Val Pro Gly Ile Gly Ile Gln Gly Pro Ala Gly Pro Pro Gly
        195                 200                 205

Pro Pro Gly Arg Phe Gly Pro Pro Gly Pro Pro Gly Pro Pro Gly Pro
    210                 215                 220

Pro Gly Pro Cys Cys Gly Tyr Ile Pro Glu Ala Pro Arg Asp Gly Gln
225                 230                 235                 240

Ala Tyr Val Arg Lys Asp Gly Glu Trp Val Leu Leu Ser Thr Phe Leu
                245                 250                 255

<210>  86
<211>  197
<212>  PRT
<213>  Artificial Sequence

<220>
<223>  HA-SP-3F-GtCLD

<400>  86

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr

35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Gly Pro
65                  70                  75                  80

Thr Gly Met Ser Gly Pro Gln Gly Leu Pro Gly Pro Thr Gly Pro Ala
                85                  90                  95

Gly Gln Asn Gly Ser Pro Gly Ser Pro Gly Ala Met Gly Pro Gln Gly
            100                 105                 110

Pro Met Gly Pro Arg Gly Tyr Arg Gly Leu Arg Gly Pro Pro Gly Asp
        115                 120                 125

Gln Gly Arg Pro Gly Asp Thr Gly Arg Pro Gly Ala Pro Gly Ile Pro
    130                 135                 140

Gly Gln Ile Gly Gln Arg Gly Pro Ile Gly Pro Met Gly Val Gln Gly
145                 150                 155                 160

Pro Glu Gly Pro Arg Gly Asn Pro Gly Leu Pro Gly Pro Pro Gly Ala
                165                 170                 175

Pro Gly Val Pro Gly Ile Gly Ile Gln Gly Pro Ala Gly Pro Pro Gly
            180                 185                 190

Pro Pro Gly Arg Phe
        195

<210>  87
<211>  172
<212>  PRT
<213>  Artificial Sequence

<220>
<223>  HA-SP-3F-FX-APRO-F-ELP4

<400>  87

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Asp Tyr Lys Asp
    130                 135                 140

Asp Asp Asp Lys Val Gly Val Ala Pro Gly Val Gly Val Ala Pro Gly
145                 150                 155                 160

Val Gly Val Ala Pro Gly Val Gly Val Ala Pro Gly
                165                 170

<210>  88
<211>  67
<212>  PRT
<213>  Artificial Sequence

<220>
<223>  3F-1F-ELP4

<400>  88

Asp Tyr Lys Asp Asp Asp Asp Lys Asp Tyr Lys Asp Asp Asp Asp Lys
1               5                   10                  15

Asp Tyr Lys Asp Asp Asp Asp Lys Gly Ser Asp Tyr Lys Asp Asp Asp
            20                  25                  30

Asp Lys Val Gly Val Ala Pro Gly Val Gly Val Ala Pro Gly Val Gly
        35                  40                  45

Val Ala Pro Gly Val Gly Val Ala Pro Gly Tyr Pro Tyr Asp Val Pro
    50                  55                  60

Asp Tyr Ala
65

<210>  89
<211>  193
<212>  PRT
<213>  Artificial Sequence

<220>
<223>  HA-SP-3F-FX-APRO-LGM-TV-ELPE4

<400>  89

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Glu Asn
145                 150                 155                 160

Leu Tyr Phe Gln Gly Val Gly Val Ala Pro Gly Glu Val Gly Val Ala
                165                 170                 175

Pro Gly Glu Val Gly Val Ala Pro Gly Glu Val Gly Val Ala Pro Gly
            180                 185                 190

Glu

<210>  90
<211>  191
<212>  PRT
<213>  Artificial Sequence

<220>
<223>  HA-SP-3F-FX-APRO-LGM-EK-ELPE4

<400>  90

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Asp Asp
145                 150                 155                 160

Asp Asp Lys Val Gly Val Ala Pro Gly Glu Val Gly Val Ala Pro Gly
                165                 170                 175

Glu Val Gly Val Ala Pro Gly Glu Val Gly Val Ala Pro Gly Glu
            180                 185                 190

<210>  91
<211>  191
<212>  PRT
<213>  Artificial Sequence

<220>
<223>  HA-SP-3F-FX-APRO-LGM-EK-ELPE4

<400>  91

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Asp Asp
145                 150                 155                 160

Asp Asp Lys Val Gly Val Ala Pro Gly Glu Val Gly Val Ala Pro Gly
                165                 170                 175

Glu Val Gly Val Ala Pro Gly Glu Val Gly Val Ala Pro Gly Glu
            180                 185                 190